(19) Europäisches Patentamt
European Patent Office
Office européen des brevets

(11) **EP 4 047 363 B1**

(12) **EUROPÄISCHE PATENTSCHRIFT**

(45) Veröffentlichungstag und Bekanntmachung des Hinweises auf die Patenterteilung:
**18.09.2024   Patentblatt 2024/38**

(21) Anmeldenummer: **22158335.4**

(22) Anmeldetag: **23.02.2022**

(51) Internationale Patentklassifikation (IPC):
**G01N 33/00** $^{(2006.01)}$     **G01M 5/00** $^{(2006.01)}$
**G01N 3/20** $^{(2006.01)}$

(52) Gemeinsame Patentklassifikation (CPC):
**G01N 33/0098; G01M 5/00**

(54) **VERFAHREN UND VORRICHTUNG ZUR ERMITTLUNG DER BRUCHSICHERHEIT EINES BAUMS**

METHOD AND DEVICE FOR DETERMINING THE RESILIENCE OF A TREE

PROCÉDÉ ET DISPOSITIF DE DÉTERMINATION DE LA RÉSISTANCE À LA RUPTURE D'UN ARBRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.02.2021   DE 102021201706**

(43) Veröffentlichungstag der Anmeldung:
**24.08.2022   Patentblatt 2022/34**

(73) Patentinhaber: **Rinn, Frank**
**69124 Heidelberg (DE)**

(72) Erfinder: **Rinn, Frank**
**69124 Heidelberg (DE)**

(74) Vertreter: **Ullrich & Naumann PartG mbB**
**Schneidmühlstrasse 21**
**69115 Heidelberg (DE)**

(56) Entgegenhaltungen:
**DE-A1- 10 032 481**

- **KOIZUMI AKIO ET AL: "Evaluation of the section modulus for tree-stem cross sections of irregular shape", JOURNAL OF WOOD SCIENCE, SPRINGER SINGAPORE, SINGAPORE, vol. 52, no. 3, 1 June 2006 (2006-06-01), pages 213 - 219, XP036681285, ISSN: 1435-0211, DOI: 10.1007/S10086-005-0747-2**
- **BURCHAM DANIEL C ET AL: "Can sonic tomography predict loss in load-bearing capacity for trees with internal defects? A comparison of sonic tomograms with destructive measurements", TREES, BERLIN, DE, vol. 33, no. 3, 5 January 2019 (2019-01-05), pages 681 - 695, XP036789922, ISSN: 0931-1890, DOI: 10.1007/S00468-018-01808-Z**

**Beschreibung**

Die Erfindung betrifft ein Verfahren zur Ermittlung der Bruchsicherheit eines Baums.

Des Weiteren betrifft die vorliegende Erfindung eine Vorrichtung zur Durchführung des Verfahrens zur Ermittlung der Bruchsicherheit eines Baums.

Aus verschiedenen Gründen, sowohl wirtschaftlichen als auch ökologischen, ist es sinnvoll, insbesondere alte Stadt-, Straßen-, Garten- und Parkbäume längstmöglich zu erhalten - auch aufgrund ihres signifikanten Beitrags zur nicht nur urbanen Biodiversität, sondern eben auch Luft- und Lebensqualität. Die vielfältige Bedeutung der Biodiversität wurde in den letzten Jahren nicht nur einer breiteren Öffentlichkeit, sondern auch der Politik bewusster, sodass es mittlerweile in vielen Ländern sogar gesetzliche, normative oder amtliche Vorschriften gibt, urbane Bäume längstmöglich zu erhalten.

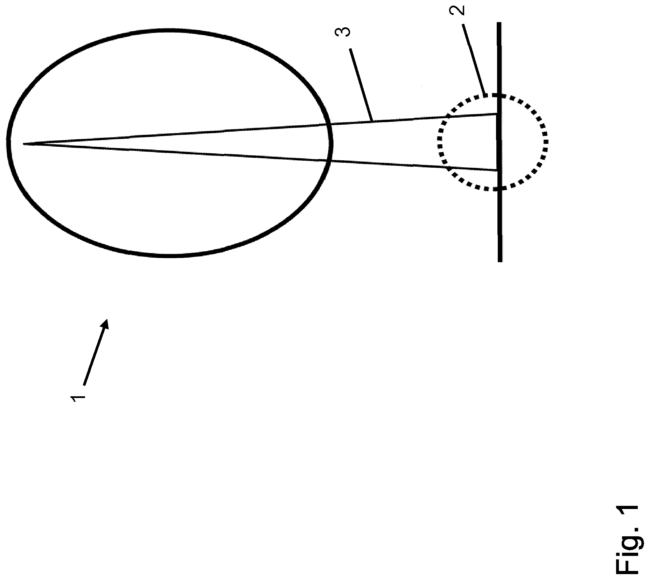

Alte Bäume, insbesondere im urbanen Umfeld, haben jedoch oft "Defekte", beispielsweise aufgrund von Wurzelkappungen, Bodenverdichtung, Rindenverletzungen, unfachlichen Rückschnitten oder sogenannten Baustellen- und Anfahrschäden, siehe Fig. 1, bei denen zumeist Fahrzeuge an Bäume stoßen und diese (lokal) beschädigen. Solche Beschädigungen beschleunigen das Eintreten und Ausweiten des (allerdings natürlicher- und notwendigerweise vorkommenden) Befalls der Bäume durch Pilze, die die Aufgabe haben, die Biomasse der Bäume letztlich wieder in den Stoffkreislauf zurückzuführen. Im Wald ist dies ein normaler Vorgang, im Wohnumfeld und an Straßen führen die vorgenannten Schäden aber zu einem beschleunigten Befall und oft zu einer vorzeitigen Gefährdung der Bruch- und/oder Standsicherheit ("Verkehrssicherheit") der betroffenen Bäume. Daher stellen solche geschädigten Bäume ein entsprechendes Risiko für Menschen und Sachwerte dar. Sobald entsprechende Schäden offenkundig sind, müssen die Baum-Eigentümer aufgrund gesetzlicher oder normativer Vorgaben die "Verkehrssicherheit" regelmäßig überprüfen. Dabei ist jeweils zu entscheiden, ob ein Baum trotz der sich entwickelnden Schäden stehen bleiben kann, die Krone zwecks Reduktion der Windlast reduziert oder der Baum gar entfernt und ersetzt werden muss.

Zur Beurteilung, ob ein Baum insbesondere aufgrund von Schäden eine signifikant erhöhte Bruchwahrscheinlichkeit aufweist, werden international bislang vor allem zwei Methoden verwendet:

- Seit ca. 1993: Visual Tree Assessment ("VTA") entwickelt vor allem von Dr. Claus Mattheck, Karlsruhe.
- Seit ca. 1997: Statisch Integrierte Abschätzung ("SIA"), entwickelt vor allem von Dr. Lothar Wessolly, Stuttgart.

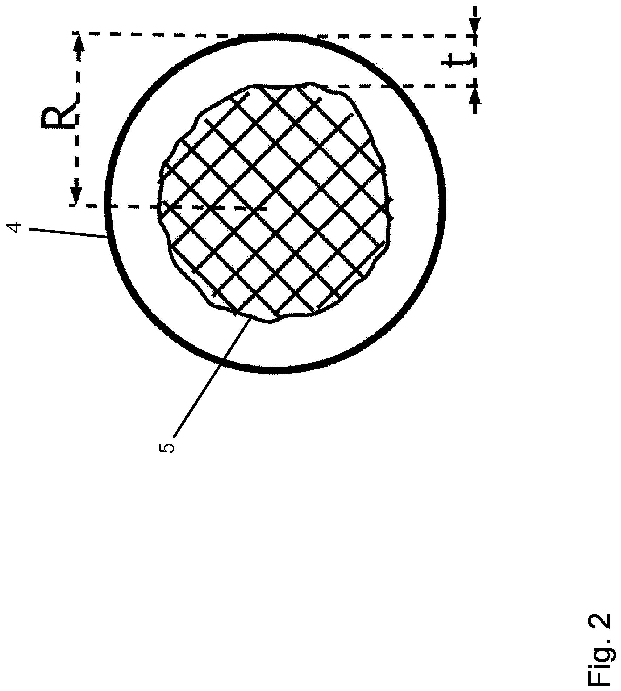

Das wichtigste und weltweit von zehntausenden Fachleuten seither angewendete VTA-Bruchsicherheits-Kriterium lautet: der Quotient aus der Dicke der äußeren intakten Wandung (t), meist "Restwandstärke" genannt, und lokalem Radius (R) sollte gemäß VTA bei "vollbekronten" Bäumen mindestens 1/3 betragen (Fig. 2) für ausreichende Bruchsicherheit. Liegt der Quotient t/R darunter, seien Maßnahmen erforderlich, um beispielsweise die Windlast zu reduzieren.

Diese sogenannte "Ein-Drittel-Regel" wurde aus Beobachtungen vor allem an noch in die Höhe wachsenden, also relativ jungen, forstlichen Bestandsbäumen mit zumeist weitgehend rundem Stamm-Querschnitt und zentrisch mittig vorliegender Fäule (Fig. 2) abgeleitet und dann auch auf Straßen- und Park-Bäume übertragen. Diese Übertragung hat sich jedoch aus verschiedenen Gründen als falsch herausgestellt:

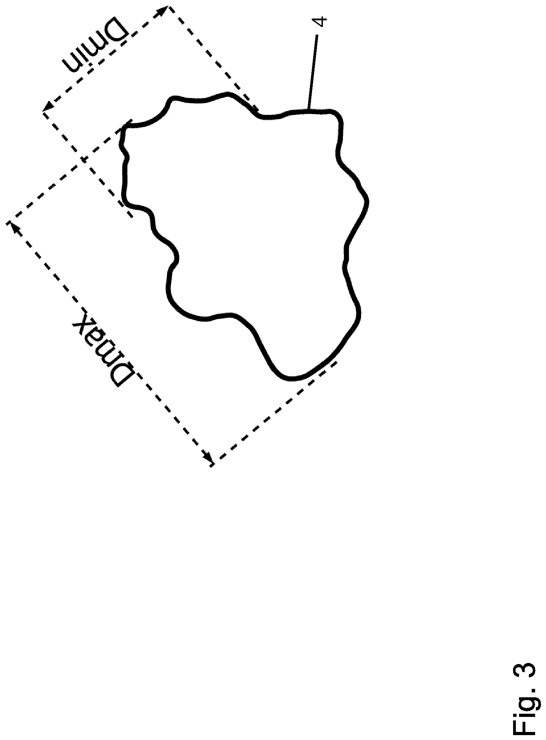

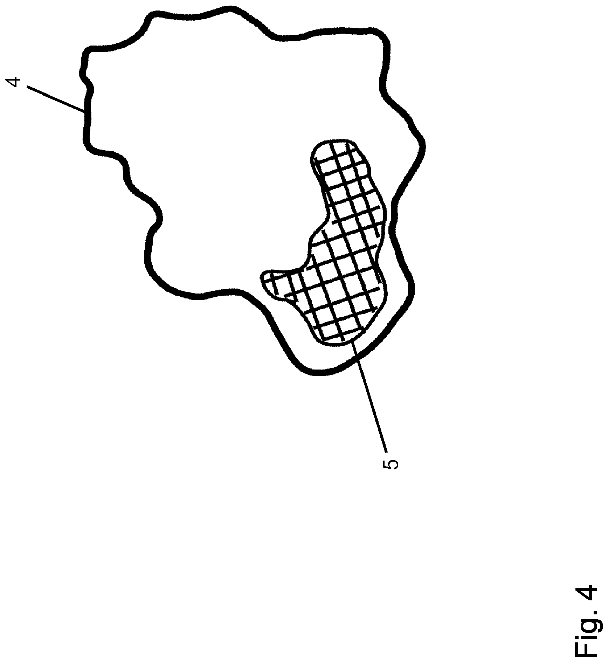

- Beim typischen Stadtbaum ist diese "1/3-Regel" schon deswegen nicht anwendbar, weil die unteren und zumeist kritischen Stammquerschnitte in der Regel nicht kreisrund sind (Fig. 3). Weil die Tragfähigkeit aber vor allem vom jeweiligen Durchmesser in Belastungsrichtung zur Dritten Potenz abhängt, spielen lokale Durchmesserabweichungen eine große, nicht nur rechnerisch dominante Rolle. Nicht-runde Querschnitte verhalten sich also biomechanisch ganz anders als runde und können daher nicht mit der vereinfachten Formel (t/R>1/3) charakterisiert werden.
- Bei den typischen Straßen- und Stadtbäumen liegen die pilzbedingten Fäulen nicht (wie im Forst meist üblich) im Zentrum der Querschnitte vor, sondern eher am Rand (Fig. 4) - weil sie zumeist durch seitliche Beschädigungen am Stamm (z.B. Anfahrschäden) oder an Wurzeln (Kappungen bei Schacht- und anderen Bodenarbeiten) entstehen. Bei solchen (zudem in der Regel unrunden) Querschnitten ist der Quotient t/R demnach grundsätzlich kein Maß für die Bruch-sicherheit, zum einen, weil es je nach Winkelrichtung hunderte verschiedene t/R-Verhältnisse gibt, zum anderen, weil selbst das geringste t/R-Verhältnis je nach Lage und sonstiger Situation keinerlei Aussage über die Tragfähigkeit des Querschnitts insgesamt erlaubt. Dies ist beispielsweise schon daran zu erkennen, dass es unzählige Bäume mit schadensbedingt offenen Querschnitten (also mit segmentweise t/R=0) sogar an Starkwind-Standorten gibt, die über Jahrzehnte hinweg unzählige Stürme mit extremen Windstärken überstehen, während benachbarte und intakte Bäume brechen. Mit segmentweise t/R=0 sind diese Bäume zumindest in diesem Bereich deutlich unter dem" Grenzwert" (t/R>1/3!), aber sie erweisen sich oft als sicher, sogar gegen Orkane. Dies zeigt, dass das t/R-Verhältnis bei dieserart Querschnittformen und Schadensverteilung kein Maß für die Bruchsicherheit darstellen kann.

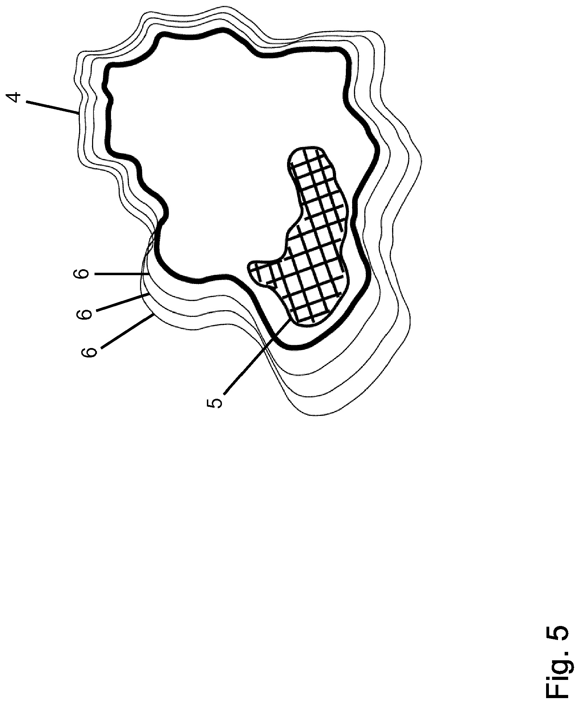

- Außerdem bilden Bäume, sobald eine signifikante Schwächung der mechanischen Tragfähigkeit in einem Querschnitt vorliegt und die am Stammrand liegenden Zellen einer höheren Belastung ausgesetzt sind, zusätzliche Zellen am äußeren Stammrand (Fig. 5), um die mechanische Tragfähigkeits-Schwächung auszugleichen ("Thigmo-Morphogenesis"). Das wiederum verändert den Durchmesser, erhöht die Tragfähigkeit und verringert die Bruchgefahr im gesamten Querschnitt.
- Schließlich wird der weiter unten beschriebene Alterseffekt von VTA nicht berücksichtigt, der dazu führt, dass alte Bäume trotz größerer Schäden bruchsicherer sein können, als junge intakte.

Die oben genannte 1/3-Regel (t/R>1/3) ist also keine wirkliche Hilfe bei der Beurteilung der Bruchsicherheit der typischen alten urbanen Straßen- und Parkbäume.

Auch aufgrund dieser Mängel im VTA-Konzept entstand die SIA-Methode und etablierte sich über die Jahre hinweg als weltweit bevorzugte Alternative bei Wissenschaftlern und Sachverständigen: bei SIA werden konkrete Materialkennwerte und biomechanische Eigenschaften von Bäumen (insbesondere E-Moduli E, kritische Dehnung e, Windwiderstandsbeiwert Cw, Resonanz- und Böenfaktoren) verwendet, um sowohl die Windlast als auch die Tragfähigkeit der (nicht nur geschädigten) Querschnitte zu berechnen und daraus Sicherheitsfaktoren zu ermitteln:

$$\text{Sicherheit} = \text{Tragfähigkeit} / \text{Belastung}$$

Eine fachlich neutrale Überprüfung der SIA-Berechnungen zeigt jedoch, dass diese Methode sowohl bei jungen als auch bei alten Bäumen zu völlig unsinnigen Ergebnissen führt. Dies liegt unter anderem daran, dass die aus der Berechnung des Verhaltens isotroper Materialien (z.B. Aluminiumrohre) unter statischer Belastung stammenden SIA-Berechnungen des Widerstandsmoments als Basis der Querschnitt-Tragfähigkeit nicht zum an-isotropen Holz mit unzähligen verschiedenen "Poisson-Verhältnissen" passen. Siehe hierzu Spatz, H.C.; Niklas, K.J. (2013): Modes of failure in tubular plant organs. Am. J. Bot. 100(2):332-336, worin die Bedeutung der holzspezifischen Anisotropie für die Tragfähigkeit hohler Querschnitte beschrieben wird. Im Ergebnis können die SIA-Ergebnisse prinzipiell keine Grundlage für eine verlässliche Beurteilung sein.

Mit VTA und SIA kann die Bruchsicherheit geschädigter Stämme von Bäumen demnach also nicht korrekt ermittelt werden. Daher werden unzählige (insbesondere urbane) Bäume unnötigerweise gekappt, zu stark zurückgeschnitten oder gar gefällt. Dies führt wiederholt zu einem erheblichen Verlust an wichtigen (nicht nur urbanen) Habitaten und damit auch an Biodiversität, darüber hinaus zu unnötig überhöhten Kosten und Mehrausgaben für die Baumpflege, sodass wiederum weniger Geld für Neu-Anpflanzungen zur Verfügung steht - denn, wenn alte Bäume längstmöglich erhalten werden, dann ist dies nicht nur ökologisch sinnvoll, sondern es senkt sogar die Gesamtkosten für das urbane Grün.

Insofern besteht also weltweit ein Bedarf nach einer Methode zur Ermittlung und nachvollziehbaren Beurteilung der Bruchsicherheit von Straßen-, Garten- und Park-Bäumen, die jedoch nicht nur wissenschaftlich korrekt ist, sondern auch einfach anwendbar, verständlich und nachvollziehbar für die in der Praxis mit der Aufgabe der Baumkontrolle befassten Personen. Denn, diese Personen müssen mit einer solchen Methode nicht nur täglich arbeiten, sondern ihre Entscheidungen nachvollziehbar dokumentieren und im Zweifelsfall oder bei Unfällen auch erklären und rechtfertigen können.

Wenn im nachfolgenden Text von "Bäumen" die Rede ist, geht es in der Regel insbesondere um ältere Straßen-, Garten- und Parkbäume, die zumeist im Wohn- und Lebensumfeld von Menschen stehen.

Des Weiteren sind aus KOIZUMI AKIO ET AL: "Evaluation of the section modulus for tree-stem cross sections of irregular shape", JOURNAL OF WOOD SCIENCE, Bd. 52, Nr. 3, 1. Juni 2006 (2006-06-01), Seiten 213-219, XP036681285, ISSN: 1435-0211, DO I : 10.1007/S 10086-005-0747-2 ein Verfahren und eine Vorrichtung zur Ermittlung der Bruchsicherheit eines Baums bekannt, wobei ein Festlegen eines zu beurteilenden Stamm-Querschnitts sowie seiner Form mit eventuell vorhandenen Schädigungen beschrieben ist.

Der vorliegenden Erfindung liegt die Aufgabe zugrunde, ein Verfahren zur Ermittlung der Bruchsicherheit eines Baums sowie eine entsprechende Vorrichtung zur Durchführung des Verfahrens anzugeben, wonach eine besonders einfache und zuverlässige Bewertung der Bruchsicherheit eines Baums mit konstruktiv einfachen Mitteln ermöglicht ist.

Erfindungsgemäß wird die voranstehende Aufgabe durch ein Verfahren mit den Merkmalen des Anspruchs 1 sowie durch eine Vorrichtung mit den Merkmalen des Anspruchs 10 gelöst.

In erfindungsgemäßer Weise ist erkannt worden, dass eine zuverlässige Bewertung der Bruchsicherheit eines Baums auf Basis lediglich einfach zu ermittelnder oder zu erfassender geometrischer Größen möglich ist. Die erfassten Werte im Hinblick auf den Stamm-Querschnitt des Baums bilden in geschickter Weise die Basis für eine einfache Abschätzung einer relativen richtungsabhängigen Tragfähigkeit des Stamm-Querschnitts über Widerstandsmomente. Komplizierte und mit extremen Fehlerschwankungen und unvermeidlichen Unwägbarkeiten belastete Windlastanalysen sowie Materialuntersuchungen oder Bestimmungen von Materialeigenschaften sind hierfür nicht erforderlich. Aufgrund der vergleichenden Relativbetrachtung zwischen eventuell geschädigtem Stamm-Querschnitt und ungeschädigtem Stamm-Querschnitt mittels einer Division fallen komplizieret zu ermittelnde Größen bei der Berechnung aus der Betrachtung heraus. Die berechneten prozentualen relativen richtungsabhängigen Tragfähigkeiten führen in ihrer Zusammenschau zu einer sicheren Bewertung der relativen Bruchsicherheit des Baums.

Folglich sind mit dem erfindungsgemäßen Verfahren und der erfindungsgemäßen Vorrichtung ein Verfahren sowie eine Vorrichtung bereitgestellt, wonach eine besonders einfache und zuverlässige Bewertung der Bruchsicherheit eines Baums mit konstruktiv einfachen Mitteln ermöglicht ist.

Im Hinblick auf ein besonders flexibles und einfaches Erfassen der erforderlichen Messgrößen kann das Erfassen des größten und des kleinsten Durchmessers ein Messen des größten und des kleinsten Durchmessers am Baum oder ein Beschaffen des größten und des kleinsten Durchmessers aus einer Datenbank umfassen. Eine Messung am Baum kann beispielsweise innerhalb weniger Minuten mittels einer Kluppe erfolgen.

Hinsichtlich einer möglichst umfassenden und zuverlässigen Ermittlung der Bruchsicherheit kann das Berechnen der Tragfähigkeit entlang mehreren Richtungen in vorgebbaren Winkelabständen entlang eines gesamten Umfangs des Stamm-Querschnitts erfolgen. Je kleiner die Winkelabstände sind, desto genauer wird die Betrachtung.

Ganz grundsätzlich kann die Tragfähigkeit in einer jeweiligen Richtung über Widerstandsmomente mittels einer Integration über eine Querschnittsfläche des Stamm-Querschnitts berechnet werden. Dies stellt eine sichere und zuverlässige Methode zur Ermittlung der Tragfähigkeit dar.

Insbesondere im Hinblick auf eine sichere Abschätzung der Bruchsicherheit mit hoher Sicherheitsreserve kann bei der Bewertung der Bruchsicherheit ein Maximalwert einer richtungsabhängigen relativen - vorzugsweise prozentualen - Schwächung der Tragfähigkeit berücksichtigt werden, um eine relative Resttragfähigkeit RQT zu bestimmen.

Hinsichtlich eines besonders aussagekräftigen Bewertungsmaßstabs und damit einer sicheren Bewertung der Bruchsicherheit des Baums kann bei der Bewertung der Bruchsicherheit eines Stamm-Querschnitts eine Grundsicherheit des Stamm-Querschnitts berücksichtigt werden. Die Bewertung der Bruchsicherheit erfolgt in diesem Fall immer in Bezug auf die normalerweise vorhandene Sicherheit eines unbeschädigten Baums, der üblicherweise, unter normalen Standbedingungen und Umweltbedingungen als sicher gilt. Die ermittelte Bruchsicherheit kann zu einem höheren Sicherheitswert als die Grundsicherheit oder zu einem niedrigeren Sicherheitswert als die Grundsicherheit führen.

Bei Vorhandensein eines noch unbeschädigten Stamm-Querschnitts, der beispielsweise keine faule und/oder hohle Stelle im Querschnitt aufweist, erfolgt bei der Bewertung der Bruchsicherheit ein Vergleich des zu beurteilenden Stamm-Querschnitts mit einem solchen intakten Stamm-Querschnitt am gleichen Stamm des Baumes, um eine relative Grundsicherheit RGS zu bestimmen. Falls eine solche relative Grundsicherheit RGS ermittelbar ist, so kann diese mit einer relativen Resttragfähigkeit RQT multipliziert werden, um eine besonders aussagekräftige relative Bruchsicherheit bereitzustellen.

Alternativ oder zusätzlich kann bei der Bewertung der Bruchsicherheit eine Reduktion einer Höhe H und/oder ein altersbedingter Rückgang einer Höhe H des Baums berücksichtigt werden, wobei sich die Bruchsicherheit hierbei um den Faktor $(H_{vorher})^3 / (H_{neu})^3$ und/oder $(H_{max})^3 / (H_{aktuell})^3$ erhöht. Eine Reduktion der Höhe H kann beispielsweise durch einen Rückschnitt oder ein altersbedingt natürliches Abbrechen von Ästen erzeugt werden. Der altersbedingte (und natürlich vorkommende) Rückgang der Baumhöhe tritt je nach Baumtyp erst nach unter Umständen vielen Jahren auf, bei vielen Bäumen beginnt dieser Vorgang im Alter zwischen 50 und 100 Jahren. Beide Arten der Verminderung der Höhe tragen zur Erhöhung der Bruchsicherheit bei, da Windlast bei den hier entscheidenden alten Bäumen vor allem proportional zur Baumhöhe zur dritten Potenz ist. Eine reduzierte Höhe hat demnach eine entsprechend starke Wirkung auf die aktuell wirkende Windlast (für alle Querschnitte des Stammes).

Weiter alternativ oder zusätzlich kann bei der Bewertung der Bruchsicherheit ein altersbedingter radialer Zuwachs des Durchmessers D des Stamm-Querschnitts des Baums berücksichtigt werden, wobei sich die Bruchsicherheit hierbei um den Faktor $(D_{aktuell})^3 / (D_{Hmax})^3$ erhöht. Der Stamm jedes lebenden Baumes wird jährlich dicker. Da sich eine Zunahme des Durchmessers D in der dritten Potenz auf die Erhöhung der Bruchsicherheit auswirkt, trägt dieser altersbedingte Effekt ganz erheblich zur Erhöhung der Bruchsicherheit bei. Insoweit wird bei Berücksichtigung dieses Effekts die Bewertung der Bruchsicherheit noch positiver, was zu einem Vermeiden eines möglicherweise unnötigen Fällens von Bäumen führt, die eigentlich noch standsicher sind und nur aufgrund unkorrekter Bewertung gefällt werden.

Ganz grundsätzlich werden bei der Bewertung oder Berechnung der Bruchsicherheit die zeitlichen Veränderungen des Quotienten aus $D^3 / H^3$ berücksichtigt, wobei D der Durchmesser des Stamm-Querschnitts und H die Höhe des Baums ist. Letztendlich führt dessen Berücksichtigung zu einer großen Vereinfachung der Abschätzung oder Bewertung der Bruchsicherheit eines Baums.

Hieraus erklärt sich auch der Alterseffekt: sobald ein Baum seine maximale Höhe erreicht hat (typischerweise in einem Alter zwischen 50 und 100 Jahren), wächst der Stammdurchmesser weiter solang der Baum lebt und das erhöht damit automatisch jährlich die Bruchsicherheit.

Wichtige Aspekte von Ausführungsbeispielen der Erfindung werden im Folgenden weiter erläutert: Ausführungsbeispiele der neuen Methode zur Beurteilung der Bruchsicherheit sind zwar einerseits wissenschaftlich wohl begründet und nachvollziehbar, benötigen jedoch einige mathematische Operationen, die zur praktischen Anwendung eine technische Vorrichtung benötigen. Daher werden hier zunächst die Grundlagen und Berechnungen erläutert, dann die Art der Eingabe und Berechnung.

Ein zentraler Ausgangspunkt der neuen Methode ist letztlich die in den meisten Ländern ähnlich bestehende rechtliche Randbedingung: die für die Beurteilung der Bruchsicherheit von Bäumen verantwortlichen Fachleute sollen erkennen, ab wann ein Baum aufgrund von Schäden im Vergleich zum (natürlichen) "Normalzustand" (=intakte Stammquerschnitte) eine signifikant erhöhte Bruchwahrscheinlichkeit aufweist und dann entsprechende Handlungen veranlassen.

Weil die Stämme auch von intakten Bäumen insbesondere bei böigen und orkanartigen Stürmen brechen können, hat auch jeder intakte ("normale") Baum eine von vielen internen und externen Faktoren abhängige ("natürliche") Versagenswahrscheinlichkeit. Wie hoch der zugehörige Sicherheitsfaktor (= Tragfähigkeit / Belastung) von Bäumen im statistischen Mittel ist, konnte aufgrund der meist unbekannten Randbedingungen und multifaktoriellen, gegenseitigen (und oft nichtlinearen) Abhängigkeiten bislang jedoch nur sehr grob abgeschätzt werden. Schon von daher kann es bei der Beurteilung von geschädigten Bäumen also eigentlich nicht darum gehen, einen absoluten Wert der Bruchsicherheit zu ermitteln, denn mit Schäden im Stamm wird die Beurteilung noch schwieriger, als beim intakten Baum.

Die o.g. rechtliche Randbedingung macht jedoch eigentlich implizit deutlich, dass es bei der Baumsicherheitskontrolle nicht um die Bestimmung absoluter Sicherheitsfaktoren geht, sondern um relative Veränderungen: die Aufgabe lautet ja, zu erkennen, ob ein Baum (in der Regel aufgrund von Schäden) eine im Vergleich zum (intakten) Normalzustand signifikant erhöhte Versagenswahrscheinlichkeit hat. Dies impliziert ja bereits, dass es nicht um die Ermittlung absoluter Sicherheitsfaktoren geht, sondern "nur" um die Erkennung von signifikant erhöhter Bruchgefahr - insbesondere im Vergleich zum natürlichen "Normalzustand", dessen Sicherheitsfaktor allerdings bislang weder wissenschaftlich exakt ermittelt noch juristisch definiert wurde.

Die hier beschriebene neue Methode ermöglicht es nun erstmals diese rechtliche Vorgabe zu erfüllen: der "Normalzustand" und der zugehörige Sicherheitsfaktor werden hier nun erstmals klar definiert und sodann als Ausgangspunkt für alle weitergehenden Beobachtungen und Berechnungen verwendet, jedoch ohne absolute Ermittlung eben all dieser Werte, denn die Berechnungen der Sicherheitsfaktoren sind bislang stets an den o.g., unüberwindlichen Fehlerquellen gescheitert. Deshalb sind die Ergebnisse aller bisherigen Berechnungs-Methoden zur Baumsicherheit grob falsch und unbrauchbar.

Um aus eigentlich unbestimmbaren Sicherheitsfaktoren etwas Sinnvolles berechnen zu können, bedarf es jedoch zunächst eines neuen, möglichst mit arithmetischen Formeln ausreichend präzise beschreibbaren, biomechanischen Wachstumsmodells von Bäumen. Dieses Modell muss einerseits (hier insbesondere hinsichtlich der Bruch-Sicherheit des Stammes) den "Normalzustand" klar definieren und andererseits erlauben, darauf aufbauend später die sicherheits-relevanten Änderungen zu erfassen und zu berechnen, die sich durch Schäden und andere Veränderungen ergeben. Dieses neue biomechanische Modell von Bäumen wiederum muss deren verschiedene Lebens- und Wachstums-Phasen berücksichtigen, denn beispielsweise die in der Forstwirtschaft etablierten Wachstums-Modelle von Bäumen beziehen sich naturgemäß auf die Bäume und Altersklassen, die in der Forstwirtschaft von Interesse sind: dort geht es insbesondere um die Produktion von Holz und daher um Bäume, die noch einen nennenswerten Holzzuwachs gewährleisten, ohne Schäden aufzuweisen, also um Bäume, die noch relativ jung sind.

Um genau diese (relativ "jungen") Bäume geht es beim Erhalt von Alt- und Habitat-Bäumen im urbanen Umfeld aber gerade nicht. Der Erhalt von geschädigten und insbesondere alten Bäumen gehörte den wirtschaftlichen Randbedingungen und forstlichen Aufgaben entsprechend bislang naturgemäß nicht zu den Schwerpunkten forstwissenschaftlicher Forschung und forstwirtschaftlicher Praxis. In den letzten Jahrzehnten ist allerdings beispielsweise die Bedeutung einer möglichst hohen Biodiversität für die Stabilität und den Wert gemischter Waldbestände auch in forstlichen Fachkreisen erkannt worden.

## Wachstumsphasen

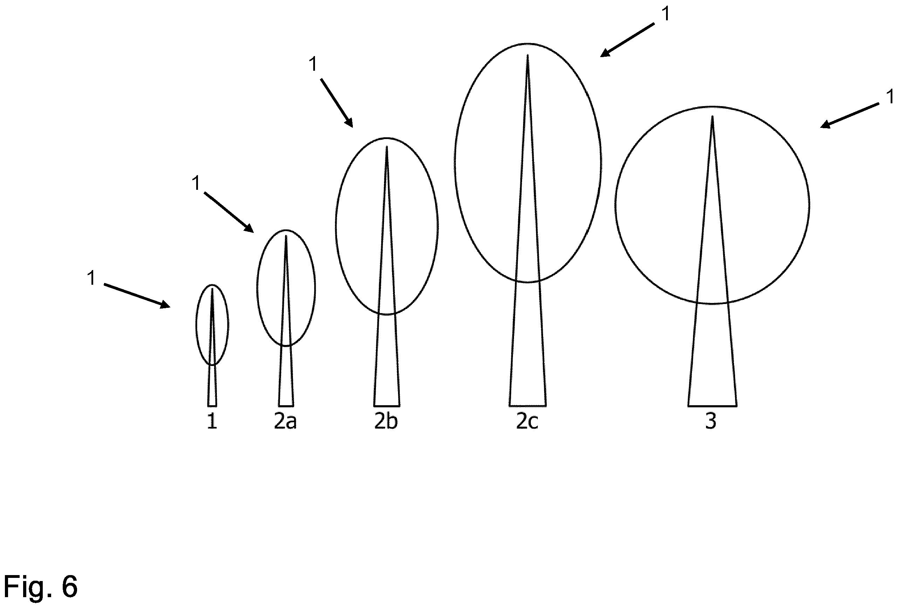

Wie in Fig. 6 zu sehen, wachsen die meisten Bäume aus dem hier relevanten biomechanischen Blickwinkel betrachtet in drei unterschiedlichen Lebens-Phasen: in den ersten Jahren ("juvenile Phase") wachsen die Bäume sehr schnell in die Höhe und sind sehr schlank. In der Natur überleben viele junge Bäume diese meist 10 bis ca. 20 Jahre dauernde Phase nicht. In der anschließenden "Explorationsphase" wachsen die verbliebenen Bäume relativ gleichmäßig in Höhe und Stammdurchmesser (der Quotient H/D bleibt ungefähr konstant) - bis sie ihre jeweils baumart-spezifisch und standort-typische, maximale Höhe erreicht haben. Anschließend werden die Bäume schrittweise kleiner, indem obere Äste im Wind abbrechen (oder geschnitten/gekappt werden) und keine solchen mehr nachwachsen. Bei manchen Baumarten bildet sich dann sogar schrittweise weiter unten eine neue Kronenstruktur aus ("Sekundärkrone"). Auch in dieser "Altersphase" nimmt der Stammdurchmesser jedoch jährlich weiter zu, solange der Baum lebt - und das hat entscheidenden Einfluss auf die Beurteilung von Schäden an solchen Bäumen.

Die bei uns und in vielen anderen Ländern, auch in den Tropen und Subtropen, im urbanen Umfeld üblichen Laubbäume erreichen typischerweise nach ca. 60 bis 80 Jahren ihre maximale Höhe, die je nach Baumart und Standort meist zwischen 20 und 30m, bei manchen auch bis ca. 40m reicht. Dann stoppt auch bei vielen Nadelbaumarten das Höhenwachstum.

Es gibt jedoch einige wenige Nadel- und sehr wenige Laubbaumarten, die auf besonderen Standorten weiter in die Höhe wachsen und bis über 100m hoch werden können. Diese seltenen Ausnahmefälle sind jedoch im Hinblick auf die hier beschriebene Problematik (Verkehrssicherheit im urbanen Umfeld) nicht relevant, weil diese Bäume dort quasi nicht vorkommen.

Das Alter des jeweils konkret zu beurteilenden Baumes ist insbesondere bei solchen Bäumen wichtig, die schon einige Jahre oder gar Jahrzehnte nicht mehr in die Höhe wachsen: weil auch die Stämme dieser (alten) Bäume jährlich dicker werden, solange sie leben, steigt damit deren Tragfähigkeit. Die im Alter zurückgehende Baumhöhe wiederum führt zu einer Reduktion der Windlast. In Kombination führt dies zu einer mitunter deutlichen Erhöhung der Bruchsicherheit über das vormals natürliche und damit "normale" Niveau hinaus, was hier nachfolgend als "Grundsicherheit" bezeichnet wird - denn die Bruchsicherheit kann zugleich durch innere Schäden wieder reduziert werden. Daher sind beide Aspekte zu kombinieren.

Auch SIA spricht von einer solchen Grundsicherheit, berechnet diese aber grundlegend falsch, was zu den vorgenannten Abweichungen von der Realität um Faktoren führt.

## Neues Biomechanik-Modell

Eine wesentliche Grundlage des neuen Biomechanik-Modells ist der seit den 1980er Jahren auch wissenschaftlich belegte Zusammenhang ("Thigmo-Morpho-Genesis"), dass der radiale Stammzuwachs verholzter Pflanzen vor allem durch die mechanische Belastung bestimmt wird:

- Werden Bäume freigestellt, indem beispielsweise benachbarte Bäume entfernt werden, so steigt die Windlast und das führt dazu, dass der jährliche radiale Stammzuwachs ansteigt, um die erhöhte Windlast zu kompensieren.
- Wenn ein Baum am Stamm verletzt wird (und Teile des Querschnitts in den Folgejahren möglicherweise durch Pilze abgebaut werden), dann steigt die mechanische Belastung der verbleibenden Zellen und auch das führt zu einem gesteigerten radialen Wachstum, um die schadensbedingte, lokale mechanische Schwächung auszugleichen.

Bei diesem radialen ("adaptiven") Wachstum sind wiederum Änderungen des Durchmessers entscheidend und wichtiger, als eine eventuelle Erhöhung der Festigkeitswerte des neu gebildeten Holzes, denn, der dominierende Faktor für die Tragfähigkeit eines Stammquerschnitts ist der Durchmesser (D): bei homogenen Materialien ist die Querschnitt-Tragfähigkeit proportional zur Festigkeit multipliziert mit dem Durchmesser zur Dritten Potenz ($D^3$). Bei inhomogenen Materialien muss ein Integral über die Querschnittfläche berechnet werden, aber auch dort bleibt am Ende der Durchmesser aufgrund der dritten Potenz der entscheidende Faktor, weil alle anderen Parameter konstant oder linear eingehen.

Insofern hat der Durchmesserzuwachs also in mehrfacher Hinsicht den dominanten Einfluss auf die Tragfähigkeit des Holzes und kann in einer speziellen, weiter unten beschriebenen Weise, als zentrales Referenzmaß für die Tragfähigkeit verwendet werden.

Die am Stamm wirkende Windlast wiederum wird insbesondere bei alten Bäumen vor allem durch die Baumhöhe (H) bestimmt und ist in erster Ordnung proportional zu $H^3$.

Diese Dominanz der Baumhöhe liegt darin begründet, dass einerseits der Winddruck proportional zur Windgeschwindigkeit zum Quadrat ist und andererseits die Windgeschwindigkeit vom Boden her kommend nach oben zunimmt, was in Kombination mit dem dabei zugleich länger werdenden Hebelarm dazu führt dass die Baumhöhe zum bestimmenden Faktor der am Stammfuß wirkenden Windlast wird.

Gleichwohl ist zu bedenken, dass dieser funktionale Zusammenhang zwischen Windlast und Baumhöhe zur dritten Potenz insbesondere bei alten, in der Höhe weitgehend ausgewachsenen oder sich bereits in der Höhe reduzierenden Bäumen der Altersphase gegeben ist. Bei jungen Bäumen, die noch vergleichsweise schnell in die Höhe wachsen, sind insbesondere die oberen Äste noch relativ flexibel und biegen sich bei steigender Windgeschwindigkeit eher weg, sodass die Kronenfläche sich im Sturm deutlich verringert. Bei diesen Bäumen liegt der o.g. Exponent der Höhe nicht exakt bei 3, sondern leicht darunter.

Allerdings ist diese Einschränkung der Gültigkeit der formelmäßigen Abhängigkeit der Windlast von der Baumhöhe (zur dritten Potenz) im hier beschriebenen Zusammenhang, der Beurteilung der Bruchsicherheit von geschädigten Bäumen, nicht relevant, weil die Bäume, die signifikante Schäden haben, in der Regel alt oder sehr alt sind - und dort gilt eben die Proportionalität zur Höhe in der dritten Potenz recht gut.

## Natürliche Modell-Bestätigung

Dass dieses neue biomechanische Modell die wichtigsten Faktoren ausreichend korrekt widerspiegelt, zeigt sich auch daran, dass die meisten Bäume nach der sogenannten juvenilen Phase (zumeist die ersten ca. 10 bis 20 Jahre) über Jahrzehnte hinweg ein nahezu konstantes Verhältnis von Höhe zu Stammdurchmesser bewahren:

$$H/D \approx konstant$$

Warum hält eine komplexe natürliche Konstruktion, wie es Bäume sind, die vielfältigen statischen und dynamischen Belastungen ausgesetzt ist, über die im Sinne der Fortpflanzung und damit der Art-Erhaltung wichtigsten Jahrzehnte hinweg einen solch zentralen allometrischen Parameter wie H/D konstant? Das muss einen wichtigen Grund haben, sonst hätte die evolutionäre Auslese dies nicht so bewirkt. Anscheinend ist dieser Parameter (H/D) also von so zentraler Bedeutung, dass sich in der bereits über 100 Millionen Jahre laufenden evolutionären Entwicklung der Baumarten genau dieses Konzept als zielführend für den Erhalt dieser Pflanzengattung erwiesen hat - sonst würde dies nicht bei fast allen Baumarten auf allen Kontinenten in ähnlicher Weise festzustellen sein.

Wenn man nun die oben beschriebenen Ansätze zur Charakterisierung von (Wind-) Belastung und Tragfähigkeit betrachtet, erklärt sich dieser Zusammenhang im biomechanischen Sinne: wenn $D^3$ die Tragfähigkeit der Stammquerschnitte bestimmt und $H^3$ die (Wind-) Belastung, dann charakterisiert der Quotient $D^3/H^3$ die Bruchsicherheit. Wenn Bäume den Quotienten H/D in ihrer artbedingt wichtigsten Lebensphase ungefähr konstant halten, dann ist auch $D^3/H^3$ konstant - und damit auch die Bruchsicherheit, sodass die Bäume damit den Fortbestand ihrer Art sichern.

Das heißt, dass bei Bäumen typische "Wachstumskonzept" (H/D $\approx$ konstant in der Explorationsphase) belegt, dass Bäume stets versuchen, einen bestmöglichen Kompromiss aus möglichst effizienter Fortpflanzung (vor allem abhängig von der Höhe des Baumes und Größe der Krone) und ausreichender Stabilität (vor allem abhängig vom Durchmesser des Stammes) zu gewährleisten.

Das in der Forschung tausendfach belegte Wachstumskonzept von Bäumen (H/D ungefähr konstant in der Explorationsphase) bestätigt damit also das hier beschriebene, neue biomechanische Modell, welches somit als Basis für die Beurteilung der Bruch-sicherheit verwendet werden kann.

Dabei ist allerdings zu bedenken, dass der absolute Wert des H/D-Quotienten eine baumart-spezifische Anpassung an den jeweiligen Standort und die dort typischen Windbelastungen darstellt: während frei stehende Bäume H/D-Werte im Bereich von typischerweise eher 30 bis 40 aufweisen, ist in engen forstlichen Beständen bei gleichen Baumarten ein H/D von oftmals 70 bis 90 zu finden. Der absolute Werte des H/D-Verhältnisses ist also nicht das entscheidende Kriterium.

## Wichtiger Aspekt der neuen Methode

Wenn also jeder Baum an seinem jeweiligen Standort einen typischen H/D-Wert ausbildet und diesen über die Jahrzehnte der Explorationsphase hinweg nahezu konstant hält, dann ist damit automatisch auch der die Bruchsicherheit charakterisierende Quotient aus $D^3/H^3$ gegeben - und damit ein hier entscheidender Referenzwert für die Beurteilung geschädigter Stamm-Querschnitte. Denn, wenn der intakte Baum mit Höhe H einen Querschnitt mit Durchmesser D bildet (und H/D konstant hält), dann repräsentiert dieser Quotient $D^3/H^3$ den natürlichen und "normalen" Sicherheitsfaktor (SFi) des intakten Baumes. Fachleute müssen dann also "nur" noch erkennen und beurteilen, ob der Sicherheitsfaktor des jeweils zu beurteilenden, geschädigten Querschnitts (SFd) niedriger oder höher ist, als der natürlich gegebene (SFi). Weil es aber nicht nur wissenschaftlich, sondern vor allem auch praktisch unmöglich ist, den Sicherheitsfaktor am Baum exakt zu bestimmen, zumal nicht zerstörungsfrei, braucht es eine andere, ganz neuartige Vorgehensweise: hierzu wird hier ein relativer Sicherheitsfaktor berechnet, indem die Sicherheitsfaktoren des defekten (SFd) durch den des (ggf. vormals) intakten (SFi) Querschnitts durcheinander dividiert werden:

$$relativer\ Sicherheitsfaktor = SFr = SFd\ /\ SFi$$

Aus dem Ansatz ergeben sich, wie nachfolgend deutlich und insbesondere auch im Vergleich zu allen bisherigen Methoden, zum einen fundamentale mathematische und methodische Vereinfachungen sowie zum anderen um Größenordnungen geringere und weniger einflussreiche Fehlerquellen.

Damit wird der aktuelle Zustand eines Baumes also nicht mit Werten aus externen Referenzdatenbanken oder baumart-bezogenen Mittelwertstudien verglichen, sondern mit sich selbst. Insofern handelt es sich also um eine spezielle Art der "Selbstreferenzierung".

Dass der Vergleich mit externen Referenzdaten nicht funktioniert, zeigt sich im Scheitern der bislang etablierten Methoden (VTA und SIA) und wird ohnehin deutlich, wenn man die beachtlich große Varianz der Materialkennwerte nicht nur zwischen Bäumen der gleichen Art im Allgemeinen, sondern ebendiese auf gleichen Standorten und sogar innerhalb eines Baumes betrachtet. Derart inhomogene und per adaptivem Wachstum an die jeweiligen lokalen Verhältnisse angepasste, komplexe mechanische Konstruktionen wie ein Baum unterscheiden sich auch aufgrund der jeweils individuell ausgeprägten Jahrringstruktur von allen anderen Bäumen (wie bei Fingerabdrücken) - auch weil die Wachstums- und Belastungsverhältnisse in der Realität urbaner Standorte sowie die genetischen Antwortmechanismen stets verschieden sind. Der einzige, der die typischen, die mittleren, wie auch extremen Windlasten am Standort eines Baumes kennt, ist der Baum selbst. Aufgrund des mechanisch adaptiven Wachstums (Thigmomorphogenesis) und der Dominanz des Durchmessers hinsichtlich der Tragfähigkeit repräsentiert daher die äußere Gestalt des Stammes eines Baumes die Summe aller bisherigen mechanisch einwirkenden Einflüsse. Besser und genauer als der Baum kann es niemand wissen. Besser und genauer als der Durchmesser zeigt es daher niemand an, welcher Lastgeschichte der Baum bis zum Zeitpunkt der Beobachtung ausgesetzt war.

Gemäß der Erfindung wird daher am Baum und an seinen äußerlich ablesbaren Gestaltänderungen abgelesen, was für die Beurteilung der Bruchsicherheit von Relevanz ist: das sind daher vor allem und zum Glück geometrische Größen, denn diese sind vergleichsweise einfach zu messen.

**Komponenten und konkrete Schritte bei Ausführungsbeispielen der erfindungsgemä-βen neuen Methode:**

**Querschnitt-Tragfähigkeit**

Unabhängig von den sonstigen Randbedingungen am konkret zu beurteilenden Baum bzw. geschädigten Stamm-Querschnitt, ist vorab eine Methode vorteilhaft, die es möglichst am Baum vor Ort erlaubt, die Minderung der Tragfähigkeit durch Schäden zu ermitteln.

Weil der Durchmesser als dominanter Faktor die Tragfähigkeit eines Querschnitts bestimmt und weil die geschädigten Stammquerschnitte der typischerweise zu beurteilenden Bäume nicht kreisrund sind, muss zunächst die Querschnittform zumindest grob erfasst und skizziert werden: auch wenn dies heute mittels Smartphone und 3D-Scan-Anwendungen bereits innerhalb von wenigen Minuten auf technischem Wege möglich ist, kann es auch manuell per Kluppe - forstlicher Groß-Mess-Schieber - erfolgen. Zumindest der größte und der kleinste Durchmesser sollten dabei erfasst und die Form des Querschnitts grob skizziert werden (Fig. 3).

Diese Querschnittskizze dient zunächst als Grundlage für die Berechnung der richtungsabhängigen, relativen Tragfähigkeit des Querschnitts über ein modifiziertes Widerstandsmoment (als rein geometrische Größe für vorzugsweise jeweils alle Belastungsrichtungen). Da das axiale Widerstandsmoment proportional zum Torsionsmoment ist und hier nur relative Veränderungen betrachtet werden, gelten die entsprechenden späteren Ergebnisse über Tragfähigkeitsschwächungen durch Schäden zugleich für beide Belastungsarten: wenn das eine durch Schäden um 10% gemindert ist, ist es das andere auch. Dies ist für die Beurteilung von Bäumen besonders wichtig (und wurde von den bisherigen Methoden nicht erfasst), weil die Torsionsfestigkeit von Holz bei oft nur ca. 10% der Zugfestigkeit liegt. Dies erklärt auch, warum die meisten realen Versagensereignisse bei geschädigten Altbäumen unter dynamischen und tordierenden Belastungen geschehen.

Zur Berechnung einer absoluten Tragfähigkeit würden Werte der Zug-, Druck- und Scherfestigkeiten aller Flächenanteile des Querschnitts in einem Finite-Elemente-Modell benötigt - weil diese Materialeigenschaften aber am stehenden Baum entweder gar nicht oder nicht zerstörungsfrei zu ermitteln oder nur sehr ungenau zu schätzen sind, scheiterten bislang alle Methoden, die auf diesem Wege versucht haben, die (absolute) Tragfähigkeit zu ermitteln (und dann in Relation zu setzen zur Belastung z.B. durch Wind oder im Vergleich mit Referenzdaten). Außerdem schwanken beispielsweise E-Moduli und kritische Dehnungen innerhalb eines Stammes sogar in kurzen Abständen sehr stark (sowohl radial und tangential als auch insbesondere axial): wenn man beispielsweise am Stamm einer alten Platane (oder ähnlichen Baumarten) vom Stammfuß nach oben zur Krone wandert, können sich die für die Tragfähigkeit kritischen Materialkennwerte (E-Moduli und kritische Dehnungen) nicht nur um einige Prozent, sondern gar um Faktoren von 5 bis 10 ändern.

Von daher ist es nicht nur praktisch, sondern auch schon theoretisch unmöglich, die Tragfähigkeit der verschiedenen Querschnitte in absoluten Werten zu ermitteln - ohne den Baum bis zum Bruch zu belasten. Auch deswegen führen die bisherigen Bruchsicherheits-Beurteiliungs-Methoden (VTA/ SIA), bei denen Materialeigenschaften lokal gemessen, mit Referenzwerten verglichen und daraus Biegebruchfestigkeiten abgeleitet werden, zu keinen verlässlichen, oft sogar abstrus falschen Ergebnissen, wenn daraus Bruchsicherheiten ermittelt und beurteilt werden.

Die hier beschriebene, neue Methode erlaubt nun erstmals eine Berechnung der relativen Tragfähigkeit eines geschädigten Querschnitts für alle Belastungsrichtungen über Widerstandsmomente (WM), ohne Materialfestigkeiten, E-Moduli und kritische Dehnungen berücksichtigen zu müssen. Der erste Schritt zur Vereinfachung der nachfolgenden Berechnungen besteht nun darin, die Zielgröße (Widerstandsmoment) durch alle konstanten Faktoren zu dividieren und damit zu einem "relativen Widerstandsmoment (RWM) zu transformieren, welches für alle Belastungsrichtungen zu berechnen ist.

Hierfür erfolgt eine rein geometrische mathematische Integration über die Querschnittfläche. Sie benötigt zunächst nur den Abstand N aller tragenden Anteile bzw. Flächenelemente (x,y) des Querschnitts zur jeweiligen neutralen Achse, also zu einer Linie durch den geometrischen Schwerpunkt, senkrecht zur jeweiligen Wind- bzw. Belastungsrich-

tung (Winkel w):

$$RWM(w) = \iint N(x,y) \, dx \, dy$$

Für Aluminium und ähnlich homogene Materialien funktioniert dieser Ansatz in erster Ordnung recht gut, allerdings nicht mehr bei sehr dünnen Wandstärken, bei denen es beispielsweise zu Schalenbeulen oder Schlauchknicken kommt. Insofern ist dieser (von SIA für die Beurteilung von hohlen Bäumen verwendete) Ansatz auch bei Rohren aus homogenem Material nur eingeschränkt nutzbar.

Bei an-isotropen Materialien wie Holz sind die Material-Eigenschaften je nach Belastungsart (Zug, Druck, Torsion, Scherung) jedoch sehr unterschiedlich, was sich insbesondere in den verschiedenen, materialspezifischen und in der Fachliteratur dokumentierten Poisson-Zahlen ausdrückt, die hier demnach als lokaler Gewichtungsfaktor einzubeziehen sind. Daraus ergibt sich:

$$RWMI(w) = \iint P(x,y) * N(x,y) \, dx \, dy$$

Der auf diesem Weg errechnete Wert des spezifischen Flächen-Widerstandsmoments gilt bei unrunden Querschnitten jeweils nur für eine Belastungs- (=Wind-) Richtung (w), weil diese die Lage und Orientierung der neutralen Achse definiert. Wenn nun die Belastungsrichtung schrittweise verändert und die obige Summe jeweils erneut berechnet wird, ergibt sich bei den typischerweise unrunden Querschnitten der Stämme alter (Park- und Strassen-) Bäume in der Regel ein jeweils anderer Wert. Wenn nun diese Werte beispielsweise für jedes Grad eines Kreises um den Baum ermittelt und notiert werden, dann ergibt sich eine Liste der (relativen) Widerstandsmomente für alle diese Belastungsrichtungen. Wenn nun alle Werte dieser Liste durch den darunter befindlichen Maximalwert geteilt und vorzugsweise in Prozent angegeben werden, dann ergibt sich eine Liste der prozentualen Tragfähigkeiten für alle Windrichtungen, deren Maximum bei mindestens einem Wert 100 beträgt, womit die Belastungsrichtung gekennzeichnet ist, gegen die der Querschnitt die größte (relative) Tragfähigkeit aufweist. Dies entspricht meist der Hauptwindrichtung, der dieser Baum ausgesetzt ist.

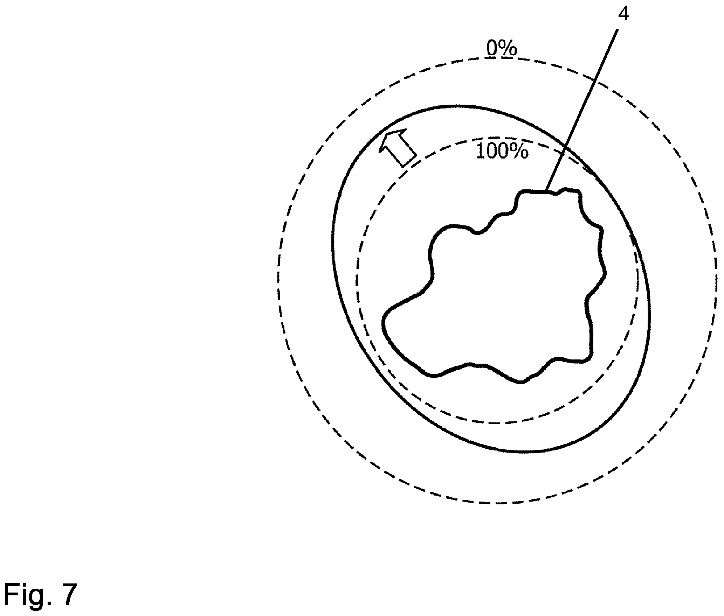

In alle anderen Richtungen ist die Tragfähigkeit entsprechend geringer und das Minimum der Prozentwerte kennzeichnet die Belastungsrichtung mit der (relativ betrachtet) geringsten Tragfähigkeit. Diese Werte könnten nun als Profil über dem jeweiligen Belastungswinkel in einem gewöhnlichen Koordinatensystem dargestellt werden, oder vorzugsweise als um den Querschnitt herumlaufende Kurve (Fig. 7). Wenn der Maximalwert (100%) beispielsweise auf dem inneren Kreis liegt, führt dies dazu, dass sich die Kurve in die Richtung am meisten nach außen 'beult', in die der Querschnitt aufgrund seiner geometrischen Form die geringste (relative) Tragfähigkeit gegen Windbelastungen aufweist, optional zusätzlich gekennzeichnet mit einem Pfeil (Fig. 7). Bei intakten Querschnitten von städtischen Bäumen zeigt dieser Pfeil in der Regel in die Richtung (w), in die es selten zu Windbelastungen kommt, also z.B. quer zur Strasse. Anhand dieser Kurve der relativen Tragfähigkeit sind also meist die örtlichen Wind- und damit Belastungsverhältnisse ablesbar, weil einerseits das Durchmesserwachstum den Belastungen folgt und andererseits vor allem der Durchmesser das mathematische Widerstandsmoment bestimmt. Der größte Durchmesser zeigt meist in Hauptwindrichtung, der geringste in diejenige, die am wenigsten Windbelastung ausgesetzt ist.

**Vom "Schaden" zur Tragfähigkeitsschwächung**

Wenn nun ein Schaden vorliegt und in der Querschnittskizze eingetragen wird, dann muss jedes Flächenelement (x,y) bei der Integration zusätzlich mit einem weiteren " Zustands-" Faktor Z(x,y) multipliziert werden, welcher seine relative lokale Tragfähigkeit hinsichtlich des Zustands kennzeichnet, beispielsweise 1 für intaktes Holz und 0 für verfaultes, entsprechende Zwischenwerte für teilweise abgebautes Holz. Hieraus ergibt sich sodann ein zustands- und geometrie-bedingtes Widerstandsmoment, welches die dominierenden Faktoren der Tragfähigkeit beinhaltet:

$$RWMD(w) = \iint Z(x,y) * P(x,y) * N(x,y) \, dx \, dy$$

Im einfachsten Falle einer manuellen, also quasi-tomographischen QuerschnittSkizze, können die tragfähigen Anteile beispielsweise in Grün (Fig. 4: umrandet und nicht schraffiert) und die nicht tragenden in Rot (hier kreuzschraffiert) gezeichnet werden. Dann können die intakten Flächenanteile mit Z(x,y)=1 und die geschädigten mit Z(x,y)=0 gewichtet werden. Wenn eine Schalltomographie erfolgt, können die Gewichtungsfaktoren auch den Farben der Tomogramme zugeordnet und entsprechend beispielsweise von 1 bis 0 abgestuft berechnet werden, womit eine präzisere Erfassung der (relativen) Tragfähigkeit erreicht wird.

Auch auf diesem Wege entsteht für alle betrachteten Belastungsrichtungen (w, z.B. für jedes Grad eines Kreises) eine Liste der jeweiligen Widerstandsmomente, die normiert wird, indem alle Werte durch das Ergebnis der vorherigen Berechnung mit Z(x,y)=1 für alle (x,y) dividiert werden: damit ergibt sich eine Kurve, die für jeden berechneten Winkel (w = Belastungsrichtung) die relative Schwächung der Tragfähigkeit des intakten Querschnitts durch die Schäden darin angibt (in Prozent).

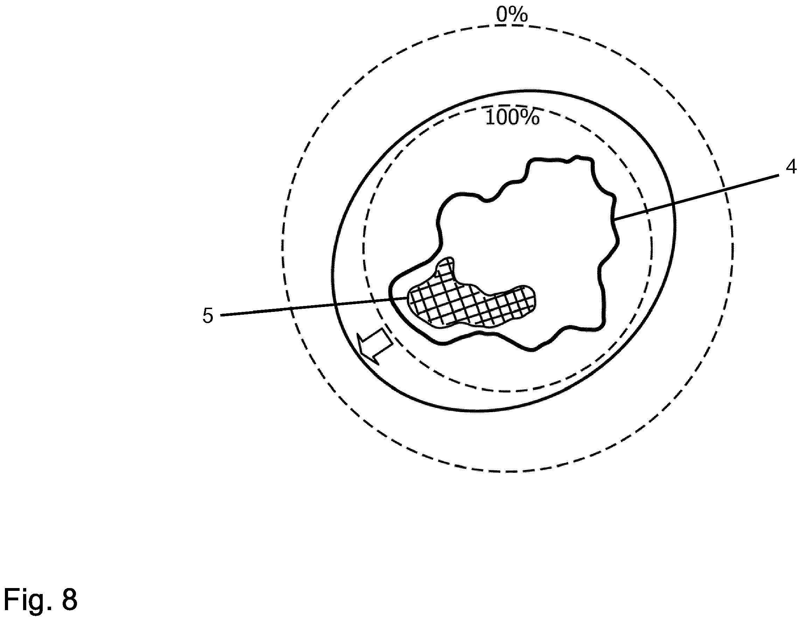

Auch diese Daten könnten beispielsweise linear in einer zweidimensionalen Graphik, aber eben auch kreisförmig um den Querschnitt herum als Kurve angezeigt werden (Fig. 8). Dies lässt die (relative!) Tragfähigkeitsschwächung durch Schäden für alle Belastungsrichtungen einfacher erkennen und beurteilen, weil sich die entsprechende Kurve am weitesten nach außen ausbeult, wohin die stärkste Schwächung zeigt. Ein Pfeil in diese Richtung kann die Erkennung dieser "schwächsten" Richtung erleichtern (Fig. 8).

Das wichtigste Ergebnis dieser Analyse ist also die relative (prozentuale) Tragfähigkeitsschwächung für alle Belastungsrichtungen sowie insbesondere ihr maximaler Wert und dessen Richtung (Fig. 8) - denn dies ist dann die potentiell gefährlichste Wind- bzw. Belastungs-Richtung für diesen betrachteten Querschnitt und ist bei weiteren Überlegungen zu berücksichtigen. Die verbliebene (relative!) Resttragfähigkeit RQT wird definiert als 100 abzüglich der hier bestimmten, maximalen Schwächung in Prozent.

Ob die auf diese Art bestimmte, prozentuale Schwächung der Tragfähigkeit eines Querschnitts kritisch ist, kann jedoch nicht (wie es bisherige Methoden fälschlicherweise versuchen) durch einen Vergleich mit einem Referenzwert beantwortet werden, sondern bedarf vielmehr weiterer Überlegungen: denn, ob eine Tragfähigkeits-Schwächung kritisch ist, hängt auch davon ab, wie hoch die Grundsicherheit des Querschnitts ist im Vergleich zum oben beschriebenen "Normal-Zustand" - und damit auch davon, ob der Baum beispielsweise bereits Reparaturwachstum gebildet hat, ob die Krone reduziert wurde und davon, wie alt der Baum ist.

So kann es denn beispielsweise auch sein, dass eine Tragfähigkeits-Schwächung von 20% bei einem noch in die Höhe wachsenden Baum kritisch, bei einem anderen, deutlich älteren Baum aber völlig unbedenklich ist, weil dieser eine entsprechend deutlich höhere "Grundsicherheit" aufweist.

**Fallunterscheidung**

Nachfolgend werden typischerweise in der Praxis vorkommende Fragestellungen je nach den konkreten Umständen und Randbedingungen unterschieden, weil es für jeden Fall bzw. Aspekt jeweils andere mathematische Wege zur vergleichenden, selbstreferenziellen Berechnung des relativen Sicherheitsfaktors gibt. Diese verschiedenen Methoden können je nach Anforderung auch miteinander kombiniert werden, insbesondere je nachdem, welche dieser Umstände gegeben sind:

- Am Stamm gibt es neben dem geschädigten auch noch intakte Querschnitte.
- Die Höhe des Baumes wurde entweder durch Rückschnitte und/oder Windbruch reduziert.
- Der Baum befindet sich in der Altersphase und wächst schon einige Jahre nicht mehr in die Höhe.

Die mit diesen typischen Aspekten geschädigter alter Bäume jeweils verbundenen mathematischen Operationen werden nachfolgend zwar zunächst einzeln beschrieben, können aber auch kombiniert werden.

**Selbstreferenzielle Querschnitt-Beurteilung**

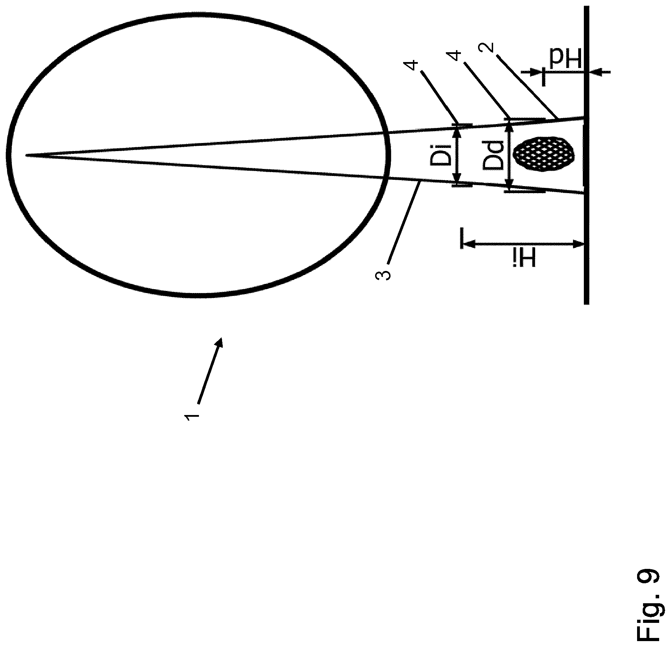

Bäume, die noch signifikant in die Höhe wachsen, sich also noch in der Explorationsphase befinden, weisen in der Regel nur punktuelle Schäden auf (z.B. nach Anfahrschäden oder Verletzungen am Stammfuß: Fig. 9), sodass sich am Stamm zumeist noch Abschnitte mit vollständig intakten Querschnitten befinden. Gemäß Sachstand der Forschung und Beobachtung sowie davon abgeleitetem, oben beschriebenem Wachstumsmodell können wir davon ausgehen, dass der Baum in einem intakten Querschnitt seinen "normalen" Sicherheitsfaktor aufrecht erhält. Diesen intakten Querschnitt nehmen wir daher nun als Ausgangs- und Referenzwert, denn er repräsentiert die insbesondere in Gerichtsverfahren wichtige "normale" Bruch-Sicherheit als Bezugsgröße (allerdings eben nur für diesen Baum). Denn, ein gesunder Baum ohne Fehler ist ja eben der 'natürliche' bzw. 'normale' und dieser hat demnach auch automatisch die natürlich gegebene Bruch-Sicherheit - die wir jedoch nicht kennen, weil die Tragfähigkeit nicht gemessenen werden kann, ohne den Baum zu zerstören und weil die reale Windlast, die an Stamm und Stammfuß wirkt, sich bislang ebenso einer ausreichend exakten Erfassung entzieht.

Das hier beschriebene, neue Konzept beurteilt nun geschädigte Querschnitte eines Stammes über einen Vergleich mit dem vorzugsweise untersten intakten Querschnitt desselben Stammes.

Weil es auch hier später nur um relative Änderungen geht, werden alle, insbesondere konstante Faktoren in den Gleichungen nicht berücksichtigt, denn diese kürzen sich bei den nachfolgenden Quotientenbildungen ohnehin heraus.

Die Windlast (WLi) am intakten Querschnitt ist in erster Ordnung gegeben durch das Biegemoment:

$$WLi = FK * Li$$

wobei FK = Kraft des Windes auf die Krone darstellt und Li = Abstand vom Kraftschwerpunkt der Krone bis zum intakten Querschnitt.

Auch hier müsste für eine absolute Erfassung der Windlast normalerweise ein Integral über die gesamte Windangriffsfläche gerechnet werden, welches jedoch von sehr vielen gar nicht, kaum oder nur sehr ungenau ermittelbaren Faktoren abhängt (Geländerauigkeit, Windgeschwindigkeits-Höhenprofil, Windwiderstandsbeiwerte, Belaubung, Temperatur, Niederschlag / Luftfeuchtigkeit, Kronengeometrie/Astlängen, ...). An den Unwägbarkeiten bei der Erfassung oder Abschätzung dieser Einflussgrößen scheitern die bisherigen (SIA-) Methoden, denn sie benötigen oder errechnen Absolutwerte der Windlast.

Für die hier beschriebene neue Methode der relativen Bewertung ist dies nicht nötig, wie sich hier nachfolgend zeigt, denn die Windlast verschwindet konzeptionsbedingt aus den Gleichungen: die Windlast am geschädigten Querschnitt (WLd) ergibt sich zu:

$$WLd = FK * Ld$$

wobei Ld = Abstand vom Kraftschwerpunkt der Krone zum defekten Querschnitt.

Die beiden Windlasten unterscheiden sich also praktisch nur in den Längen der Hebelarme (Ld zu Li), denn die (unbekannte und kaum berechenbare) Kraft auf die Krone ist ja identisch.

Die Tragfähigkeiten der betreffenden Querschnitte (TF) wiederum sind vor allem proportional zur jeweiligen Festigkeit (Q) und zum jeweiligen Durchmesser (D), letzterem aber ungefähr zur dritten Potenz:

$$TF \sim Q * D^3$$

Änderungen im Durchmesser sind also weitaus wichtiger und wirkungsvoller, als die ohnehin inhomogen verteilten und schwierig festzustellenden (absoluten) Festigkeitswerte, weil er in der dritten Potenz eingeht.

Bisherige Methoden scheitern auch in diesem Bereich, weil sie punktuelle / lokale Festigkeitseigenschaften bestimmen und daraus auf den gesamten Querschnitt oder gar auf den gesamten Stamm oder Baum schließen, was aber aufgrund der enormen natürlichen Varianz in den Materialeigenschaften (um Faktoren in geringen Abständen) zu erheblichen Abweichungen zwischen den ermittelten und realen Tragfähigkeiten führt. Daher soll hier per Selbstreferenz anders gerechnet werden.

Die Bruchsicherheit (BS) eines Querschnitts ergibt sich aus dem Quotienten zwischen Tragfähigkeit (TF) und Belastung (Windlast WL):

$$BS = TF / WL$$

Angesichts der oben beschriebenen funktionalen Abhängigkeiten ist die wichtigste Proportionalität damit gegeben durch:

$$BS \sim Q * D^3 / (FK * L)$$

D = Durchmesser des zu beurteilenden Querschnitts
FK = Kraft des Windes auf die Krone
L = Hebelarmlänge (Abstand Kronenkraftschwerpunkt zum jeweiligen Querschnitt am Stamm).

Hier kann zwecks Vereinfachung auch die Hälfte der Baumhöhe verwendet werden, was weitere Sicherheit im System belässt, weil die Längenunterschiede der Hebelarme damit etwas größer werden, als sie in Realität sind.

Die Bewertung der Bruchsicherheit des defekten Querschnitts soll hier nun nicht, wie in bisherigen Methoden üblich, durch eine Absolutwertberechnung erfolgen (Bruchfestigkeit / Windlast) oder etwa durch eine Bestimmung des Quotienten von Restwandstärke zu Radius, sondern über einen Vergleich mit einem intakten Querschnitt am gleichen Stamm, indem die Bruchsicherheit des defekten Querschnitts durch die des intakten geteilt wird:

$$BSd / BSi = [Qd * Dd^3 / (FK * Ld)] / [Qi * Di^3 / (FK * Li)]$$

Weil die Windkraft auf die Krone für alle Querschnitte am Stamm gleich ist und sich die in den Querschnitten wirkenden Biegemomente jeweils nur durch die Hebelarmlänge unterscheiden, ergibt sich eine deutlich einfachere Gleichung:

$$BSd / BSi = [Qd * Dd^3 / Ld] / [Qi * Di^3 / Li]$$

Hier ist die ohnehin schwierig bis unmöglich bestimmbare Windlast nicht mehr vorhanden, was die Mathematik entsprechend deutlich vereinfacht und Fehlerquellen reduziert. Diese Gleichung ist jedoch noch dadurch "schwierig" lösbar, dass die Festigkeitswerte (Q) der beiden Querschnitte nicht bekannt sind und quasi auch nicht zerstörungsfrei ausreichend genau messbar - zumal die Querschnitte aufgrund der Variabilität der Materialkennwerte ohnehin nicht mit einem Wert für die Festigkeit charakterisiert werden könnten. Allerdings haben alle bisherigen Untersuchungen gezeigt, dass die Festigkeitswerte gerade in den äußeren, statisch wichtigsten, kompensatorischen Stammradial-Zuwächsen um Schäden herum stets höher sind, als im sonstigen, "normalen" Holz intakter und ungestörter Querschnitte:

$$Qd > Qi$$

Wenn wir also

$$Qd = Qi$$

setzen, 'verschenken' wir quasi etwas Sicherheit in der hier beschriebenen Methode zur Beurteilung der Bruchsicherheit, vereinfachen aber die entscheidende Gleichung erheblich:

$$RGS = BSd / BSi = [Dd^3 / Ld] / [Di^3 / Li]$$

Denn, somit ergibt sich die relative ,Grundsicherheit' (RGS) des defekten Querschnitts aus rein geometrischen und am Baum in wenigen Minuten erfassbaren Grö-βen (Durchmesser, Höhen) - bezogen auf die Bruchsicherheit des intakten Querschnitts (und damit dem auch in Gerichtsverfahren als Vergleichs-Referenz wichtigen "Normal-Zustands"). Durch das "Kürzen" der Festigkeitswerte aus der Gleichung verschwindet zugleich eine der wesentlichen, kaum bestimmbaren Ursachen für die gro-βen Fehlerabweichungen bisheriger Methoden, die zumeist Festigkeitswerte aus Referenzkatalogen verwenden, die aber am konkreten Baum, zumal an der konkret zu beurteilenden Stelle selten bis nie korrekt sind, sondern um Faktoren abweichen können.

Wenn ein geschädigter Querschnitt infolge des adaptiven Kompensationswachstums beispielsweise 20% dicker ist, als der intakte Vergleichs-Querschnitt des gleichen Stammes (Dd = Di * 1.2) und ca. 10% weiter vom Windkraftschwerpunkt entfernt (Ld = Li * 1.1), dann ergibt sich:

$$RGS = BSd / BSi = (1.2)^3 / 1.1 \approx 1.57$$

und damit eine um knapp 60% höhere ,Grundsicherheit' (ohne Berücksichtigung eventueller Schäden).

Wenn der Schaden im Holz des defekten Querschnitts zu einer Schwächung seiner mechanischen Tragfähigkeit um 20% führen würde (was einem schon relativ großen Schaden entspricht), dann wäre der obige relative Grundsicherheitsquotient (RGS) noch mit der relativen Resttragfähigkeit (RQT=0.8) zu multiplizieren. Somit läge die relative Bruchsicherheit (RBS) des defekten Querschnitts unter diesen Umständen also noch immer deutlich über dem Niveau des intakten Querschnitts:

$$RBS = RGS * RQT = 1.57 * 0.8 \approx 1.25$$

Auf diesem Wege kann also die Bruchsicherheit eines geschädigten Querschnitts in wenigen Minuten am Baum mit ausreichender Genauigkeit und auf nachvollziehbare Weise bestimmt und dokumentiert werden - unter Bezug auf einen intakten Querschnitt des gleichen Stammes und somit in relativer, prozentualer Weise, ohne einen Absolutwert der Bruchsicherheit angeben zu müssen und ohne die schwierig bis unmöglich bestimmbaren Einflussgrößen (Windlast, Materialkennwerte) bestimmen oder abschätzen zu müssen, denn diese kürzen sich aus den Gleichungen heraus, sodass nur noch geometrische, einfach am Baum bestimmbare Größen übrig bleiben. Die Sicherheitsbeurteilung bezieht sich damit auf den intakten, also 'normalen' Querschnitt desselben Baumes und benötigt damit keine externe Referenzdaten, die ohnehin nie der konkret am Baum vorhandenen Belastung und Tragfähigkeit entsprechen können, denn niemand kennt die notwendigen Tragfähigkeiten besser, als der Baum selbst und dies zeigt er vor allem im jeweiligen Durchmesser des Stammes an.

**Höheneffekt**

Da Bäume den Quotienten H/D nach der juvenilen Phase ungefähr konstant halten (weil damit auch die Bruchsicherheit $D^3/H^3$ ungefähr konstant bleibt), bis sie ihre maximale Höhe erreicht haben und weil die Windlast ungefähr proportional zur Baumhöhe (H) zur dritten Potenz ist, bedeutet jede Reduktion der Höhe (durch Rückschnitte und/oder durch Ast-Abbrüche in Stürmen) nicht nur eine entsprechend deutliche Reduktion der Windlast, sondern zugleich auch eine Erhöhung der Bruchsicherheit um den Faktor

$$(H_{vorher})^3 / (H_{neu})^3$$

wobei $H_{neu}$ die reduzierte Höhe darstellt und $H_{vorher}$ die vorherige.

Ab dem Zeitpunkt, an dem der Baum seine maximale Höhe ($H_{max}$) erreicht hat und sich seine Höhe von Natur aus langsam und schrittweise verringert (ggf. zusätzlich durch Rückschnitte), nimmt die Windlast entsprechend ab, was automatisch zu einem entsprechenden Anstieg der Bruchsicherheit um diesen Faktor führt:

$$(H_{max})^3 / (H_{aktuell})^3$$

Viele Behörden (weltweit!) führen Baumkataster und notieren darin nicht nur den Zustand, sondern insbesondere auch den Stammdurchmesser (D) wie auch die Gesamthöhen (H) zum Zeitpunkt der jeweiligen Inspektionen. Dass diese beiden geometrischen ("allometrischen") Größen traditionell aufgezeichnet werden, liegt natürlich auch am Einfluss der Forstwirtschaft auf die Baumpflege.

Von daher gibt es also oftmals Durchmesser- und Höhen-Daten, die für die Berechnung des hier beschriebenen "Höheneffekts" verwendet werden können. Wenn keine solchen Daten vorliegen, dann können jedoch Abschätzungen aufgrund von Erfahrungswerten erfolgen: für die meisten Straßen- und Parkbaumarten ist denn auch recht gut bekannt, wann sie welche maximale Höhe erreichen, zumal diese Angaben auch von den Baumschulen angegeben werden, weil diese Eigenschaften wichtig und zu beachten sind beim Einkauf der standortgemäß am besten geeigneten Baumart und Variante.

Von daher kann die Höhenentwicklung bei quasi allen urbanen Bäumen, die (aufgrund von Schäden) hinsichtlich ihrer Bruchsicherheit zu beurteilen sind, entweder auf Basis gemessener Daten oder mittels fachlicher Abschätzung erfolgen, um damit die Auswirkung auf die Bruchsicherheit zu ermitteln.

**Alterseffekt**

Sobald ein Baum seine maximale Höhe erreicht hat ($H_{max}$, $D_{Hmax}$), geht das radiale (Dicken-) Wachstum des Stammes weiter, solange der Baum lebt, weil Bäume jedes Jahr einen neuen "Jahrring" bilden, insbesondere auch, um darin Wasser zur Krone zu leiten. Weil die Höhe aber nicht mehr ansteigt, steigt auch die Windlast nicht mehr, sodass mit jedem radialen Durchmesserzuwachs auch die Bruchsicherheit des Stammes ansteigt:

$$(D_{aktuell})^3 / (D_{Hmax})^3$$

Auch hier sind die in den Baumkatastern enthaltenen Daten (Durchmesser, Höhe, ... ) hilfreich, wenn dieser Alterseffekt für einen alten Baum abgeschätzt werden soll. Falls keine Daten vorhanden sind, kann die Durchmesserentwicklung eines beispielsweise schon ca. 30 Jahren nicht mehr in die Höhe wachsenden Baumes abgeschätzt werden. Denn, für die meisten Baumarten ist bekannt, wie sich der Durchmesser insbesondere in der Altersphase entwickelt. Auf diese Weise kann der "Alters-Effekt", also der Gewinn an Grundsicherheit aufgrund des steigenden Durchmessers ermittelt werden.

**Praktische Anwendung:**

**Werkzeuge und Hilfsmittel**

Um das hier beschriebene, neue Verfahren praktisch anwenden zu können, braucht man normalerweise zunächst etablierte "Werkzeuge" wie beispielsweise eine Kluppe (Mess-Schieber für Bäume) und einen Baumhöhen-Messer, um die für die hier beschriebenen Berechnungen notwendigen äußeren Parameter (Durchmesser und Höhen) erfassen zu können; sodann eine Vorrichtung, in die diese Daten eingegeben und entsprechend miteinander verrechnet werden können. Benötigt wird dann noch eine Möglichkeit, um die Form des zu beurteilenden Querschnitts zu erfassen und das Zustands- bzw. Tragfähigkeits-Tomogramm zu erstellen, denn dies ist ja die Basis für die Berechnung der relativen Schwächung der Tragfähigkeit. Weil der innere Zustand, insbesondere die Ausdehnung der Schäden in den meisten Fällen von au-βen ohne technische Hilfsmittel nicht erkennbar ist, kommen hier oft Messgeräte zum Einsatz, beispielsweise Bohrwiderstandsmessungen (Rinn 1990, ...) und Schalltomographie (Rinn 1999).

Wird ein tragbarer Computer mit vorzugsweise eingebauter oder alternativ anschließbarer Kamera sowie Eingabemöglichkeiten für die vorgenannten Parameter und Anschlussmöglichkeiten für externe Messgeräte verwendet, können alle diese Aufgaben mit einem Gerät ausgeführt werden, was nicht nur vielfach effizienter ist, sondern auch Fehlerquellen bei Eingabe und Übertragung minimiert.

Mit modernen Kameras können nicht nur ausreichend hochauflösende Fotos angefertigt werden, sondern Objekte darauf auch mit hier ausreichender Präzision vermessen werden. Auf diesem Wege können Baum-Höhen und Stamm-Durchmesser sowie auch die Form, Position, Ausrichtung und Abmessungen von Querschnitten vergleichsweise schnell erfasst und dokumentiert werden.

Nach Eingabe der anderen, für die hier beschriebene Berechnung notwendigen Parameter, kann der tragbare Computer die Einzelergebnisse und das Gesamtergebnis berechnen, anzeigen, speichern und übertragen.

**Konkreter Ablauf gemäß einem vorteilhaften Ausführungsbeispiel des Verfahrens:**

1 Querschnitt

1.1 Feststellung des zu beurteilenden Stamm-Querschnitts, zumeist geht es um den am stärksten geschädigten.
1.2 Messung und Eingabe zumindest des kleinsten und größten Durchmessers des zu beurteilenden Querschnitts. Die weiteren am Baum zu bestimmenden Durchmesser sind in der Richtung des hier größten Durchmessers zu bestimmen.
1.3 Zeichnen bzw. Skizzieren der Querschnittform inkl. geschädigter Anteile und Nordrichtung.
1.4 Berechnung der relativen, richtungsabhängigen Tragfähigkeiten sowie ihrer Schwächung.

2 Sofern vorhanden: Ermittlung der Höhe und des Durchmessers des untersten noch intakten Querschnitts des Stammes sowie Eingabe der entsprechenden Werte.
3 Alter

3.1 Aktuelles Alter (A) des Baumes ermitteln oder schätzen und eingeben.
3.2 Einordnung des Baumes in eine der relevanten Lebensphasen: Explorations-Phase oder Altersphase.
3.3 Falls sich der Baum in der Altersphase befindet:

3.3.1 Eingabe der Anzahl der seit Erreichen der maximalen Höhe vergangenen Jahre (JP).
3.3.2 Durchmesser des Stammquerschnitts zum Zeitpunkt der Erreichung der maximalen Baumhöhe ($D_{Hmax}$) eingeben oder aus der Wachstumsrate und der Anzahl der Jahre zurückrechnen.

4 Baumhöhe (H)

4.1 Eingabe der aktuellen Baumhöhe (Ha).
4.2 Falls sich der Baum schon in der Altersphase befindet: Eingabe der ehemals größten Höhe (Hmax).

5 Kombinierte Verrechnung der eingegebenen Parameter.

**Neben-Anwendungen:**

Auch wenn diese Beschreibung sich auf Schäden im Stamm von Bäumen fokussiert, sind die hier angesprochenen Aspekte zumindest zum Teil auch auf Starkäste und insbesondere auf sogenannte "Stämmlinge" anwendbar. Auch dort kann beispielsweise der Vergleich mit intakten Querschnitten des gleichen Astes oder Stämmlings zur Beurteilung erfolgen, um Schäden zu beurteilen.

Sobald ein Baum beispielsweise schief steht, kann die Gewichtskraft einen signifikanten Beitrag zur Belastung beitragen, was allerdings normalerweise aufgrund des adpativen Wachstums der Zellen ausgeglichen wird und deswegen meist nicht zu berücksichtigen ist.

Ausführungsbeispiele der vorliegenden Erfindung können ein Verfahren und eine Vorrichtung zur Ermittlung und/oder Verarbeitung von Daten und/oder Parametern hinsichtlich einer Bestimmung oder Bewertung der Bruchsicherheit von Bäumen bereitstellen.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche und andererseits auf die nachfolgende Erläuterung bevorzugter Ausführungsbeispiele der erfindungsgemäßen Lehre anhand der Zeichnung zu verweisen. In Verbindung mit der Erläuterung der bevorzugten Ausführungsbeispiele anhand der Zeichnung werden auch im Allgemeinen bevorzugte Ausgestaltungen und Weiterbildungen der Lehre erläutert. In der Zeichnung zeigen

Fig. 1    in einer schematischen Darstellung einen typischerweise zu untersuchenden Baum,

Fig. 2    in einer schematischen Darstellung einen typischen zu beurteilenden Stamm-Querschnitt,

Fig. 3    in einer schematischen Darstellung einen ungeschädigten Stamm-Querschnitt eines typischen Stadtbaums,

Fig. 4    in einer schematischen Darstellung einen geschädigten Stamm-Querschnitt eines typischen Stadtbaums,

Fig. 5    in einer schematischen Darstellung einen geschädigten Stamm-Querschnitt mit entsprechend der Schädigung entstandenen Jahresringen,

Fig. 6    in einer schematischen Darstellung eine Abfolge eines typischen Baumwachstums,

Fig. 7    in einer schematischen Darstellung eine winkelabhängige relative Tragfähigkeit um einen ungeschädigten Stamm-Querschnitt herum,

Fig. 8    in einer schematischen Darstellung eine winkelabhängige relative prozentuale Schwächung der Tragfähigkeit bei einem geschädigten Stamm-Querschnitt und

Fig. 9    in einer schematischen Darstellung einen am Stammfuß geschädigten Baum mit eingezeichneten intakten und geschädigten Stamm-Querschnitten.

Fig. 1 zeigt in einer schematischen Darstellung einen typischerweise zu untersuchenden Baum 1 mit einem einen Stammfuß 2 aufweisenden Stamm 3. Bei den typischerweise hinsichtlich ihrer Bruch- und Standsicherheit zu untersuchenden zumeist alten und urbanen Bäumen 1 liegen die zu beurteilenden Schäden meist am unteren Stamm 3 und Stammfuß 2 vor.

Fig. 2 zeigt in einer schematischen Darstellung einen typischen zu untersuchenden Stamm-Querschnitt 4. Gemäß der bekannten VTA-Methode soll der Quotient aus der Dicke der äußeren intakten Wandung t, meist "Restwandstärke" genannt, und lokalem Radius R "vollbekronter" Bäume mindestens 1/3 betragen, um eine ausreichende Bruchsicherheit zu gewährleisten. Der Stamm-Querschnitt 4 weist einen geschädigten Bereich 5 im Innern des Stamm-Querschnitts 4 auf.

Fig. 3 zeigt in einer schematischen Darstellung einen ungeschädigten Stamm-Querschnitt 4 eines typischen Stadtbaums. Beim typischen Stadtbaum sind die unteren und zumeist kritischen Stamm-Querschnitte 4 in der Regel nicht kreisrund, sondern in ihrer Form bestimmt von den Wurzelanläufen, die sich wiederum den standörtlichen Gegebenheiten entsprechend ausprägen. Dabei zeigt der größte Durchmesser Dmax meist in Hauptwindrichtung und der geringste Durchmesser Dmin zumeist in die Richtung, in der die geringste Windlast auf den Baum einwirkt.

Fig. 4 zeigt in einer schematischen Darstellung einen geschädigten Stamm-Querschnitt 4 eines typischen Stadtbaums mit einem geschädigten Bereich 5. Bei den typischen Straßen- und Stadtbäumen liegen die pilzbedingten Fäulen nicht wie im Forst meist üblich im Zentrum der Querschnitte 4 vor, sondern eher am Rand, weil sie zumeist durch seitliche Beschädigungen am Stamm, beispielsweise Anfahrschäden, oder an Wurzeln aufgrund von Kappungen bei Schacht- und anderen Bodenarbeiten entstehen.

Fig. 5 zeigt in einer schematischen Darstellung einen geschädigten Stamm-Querschnitt 4 mit geschädigtem Bereich 5 und entsprechend der Schädigung entstandenen Jahresringen 6. Sobald eine signifikante Schwächung der mechanischen Tragfähigkeit durch Schäden (kreuz-schraffierte Teilfläche als geschädigter Bereich 5) in einem Querschnitt 4 vorliegt, sind insbesondere die am nächstgelegenen Stammrand liegenden, lebenden Zellen ("Kambium") einer höheren mechanischen Belastung ausgesetzt. Diese Kambialzellen reagieren darauf durch ein verstärktes Dickenwachstum in der Wachstumsperiode, um die mechanische Tragfähigkeits-Schwächung durch "adaptiven" Zuwachs auszugleichen ("Thigmo-Morphogenesis"). Daher sind die radialen Jahreszuwächse, in Klimazonen mit einer Winterwachstumsruhe auch "Jahrringe" 6 genannt, im Bereich um Schäden herum deutlicher ausgeprägter, als in eher ungestörten Bereichen des Querschnitts 4.

Fig. 6 zeigt in einer schematischen Darstellung eine Abfolge eines typischen Baumwachstums. Aus dem hier entscheidenden, biomechanischen Blickwinkel betrachtet wachsen die typischen urbanen Bäume 1 in drei unterschiedlichen Buchung Lebens-Phasen: in den ersten Jahren ("juvenile Phase") wachsen die Bäume 1 sehr schnell in die Höhe und sind sehr schlank. In der anschließenden "Explorationsphase" (2a, 2b und 2c) wachsen die verbliebenen Bäume 1 relativ gleichmäßig in Höhe und Stammdurchmesser - bis sie ihre jeweils baumart-spezifisch und standort-typische, maximale Höhe erreicht haben. Dann folgt eine je nach Baumart und Standort unterschiedlich lange ausgeprägte Phase, in der die Baumhöhe nahezu konstant bleibt oder langsam abnimmt. Anschließend werden die Bäume 1 schrittweise kleiner, indem obere Äste im Wind abbrechen oder geschnitten/gekappt werden und keine solchen mehr nachwachsen. Bei vielen Baumarten bildet sich dann schrittweise weiter unten eine neue Kronenstruktur aus ("Sekundärkrone"), wozu der Baum 1 auch dadurch angeregt wird, dass nach dem Abbruch von Ästen Licht in das Innere der Krone gelangt, welches dort Knospen in der Rinde aktiviert, auszutreiben. Daraus entstehen dann sogenannte "Re-Iterate", die dann eine Sekundär- und manchmal auch eine Tertiärkrone bilden. Auch in dieser "Altersphase" (3) nimmt der Stammdurchmesser jedoch jährlich weiter zu, solange der Baum 1 lebt - und das hat entscheidenden Einfluss auf die Beurteilung von Schäden an solchen Bäumen 1.

Fig. 7 zeigt in einer schematischen Darstellung eine winkelabhängige relative Tragfähigkeit um einen ungeschädigten Stamm-Querschnitt 4 herum. Das für jeden Winkel (w) als Maß für die relative Tragfähigkeit gewichtete Widerstandsmoment wird hier als Prozent-Kurve um den Querschnitt 4 herum angezeigt, wobei in diesem Beispiel 100% innen und 0% außen liegt, damit sich die Kurve in die Richtung am meisten nach außen ausbeult, in die die geringste Tragfähigkeit gegen (Wind-) Biegebelastung vorliegt.

Fig. 8 zeigt in einer schematischen Darstellung eine winkelabhängige relative prozentuale Schwächung der Tragfähigkeit bei einem geschädigten Stamm-Querschnitt 4. Wenn die geschädigten Anteile eines Querschnitts 4 bei der Berechnung des gewichteten Widerstandsmoments berücksichtigt und die sich ergebende, relative Tragfähigkeit für jeden Winkel durch den Wert für den gleichen Querschnitt 4 ohne Schäden geteilt wird, dann ergibt sich eine Kurve der relativen (prozentualen) Schwächung, die hier um den Querschnitt 4 herum angezeigt wird. Wenn die Ordinate so orientiert ist, dass 100% innen und 0% außen liegt, dann beult sich diese Kurve dort am meisten nach außen aus, wohin die stärkste Schwächung vorliegt. Dies kann beispielsweise mit einem Pfeil zusätzlich gekennzeichnet werden, denn es ist die Richtung, die potentiell die höchste Bruchwahrscheinlichkeit hat.

Fig. 9 zeigt in einer schematischen Darstellung einen am Stammfuß 2 geschädigten Baum 1 mit eingezeichneten intakten und geschädigten Stamm-Querschnitten 4. Beschädigungen am Stammfuß 2 führen meist zu einer "adaptiven" Wachstumsreaktion, indem der Baum 1 im Bereich des Schadens breitere Jahrringe als am sonstigen Stamm 3 bildet, was zu einer lokalen Verdickung führt. Zur Beantwortung der Frage nach der Bruchsicherheit in diesem Bereich sind gemäß dem hier vorgestellten neuen Verfahren im Wesentlichen nur geometrische Größen erforderlich: neben der Gesamthöhe H des Baumes wird die Höhe des defekten - Hd - und die des untersten intakten - Hi - Querschnitts 4 des Stamms 3 bestimmt; außerdem noch der maximale Durchmesser des geschädigten - Dd - Querschnitts und den Durchmesser des intakten - Di - Vergleichsquerschnitts in gleicher Richtung gemessen.

Hinsichtlich weiterer vorteilhafter Ausgestaltungen des erfindungsgemäßen Verfahrens sowie der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele lediglich zur Erörterung der beanspruchten Lehre dienen, diese jedoch nicht auf die Ausführungsbeispiele einschränken.

**Bezugszeichenliste**

1   Baum
2   Stammfuß
3   Stamm
4   Stamm-Querschnitt
5   geschädigter Bereich
6   Jahresring
t   Wandung
R   Radius

**Patentansprüche**

1. Verfahren zur Ermittlung der Bruchsicherheit eines Baums (1), umfassend die folgenden Schritte:

   - Festlegen eines zu beurteilenden Stamm-Querschnitts (4) des Baums (1);
   - Erfassen mindestens eines größten und eines kleinsten Durchmessers im Stamm-Querschnitt (4) sowie seiner Form mit eventuell vorhandenen Schädigungen des Stamm-Querschnitts (4);
   - Berechnen - ausgehend von einer durch einen geometrischen Schwerpunkt des Stamm-Querschnitts (4) verlaufenden Achse - der neutralen Achse - einer prozentualen relativen richtungsabhängigen Tragfähigkeit in mehreren definierbaren Richtungen über jeweils dem Stamm-Querschnitt (4) zugehörige und gemäß Anisotropie des Materials spezifisch ermittelte Widerstandsmomente, wobei zur Berechnung einer jeweiligen prozentualen relativen richtungsabhängigen Tragfähigkeit für jede der mehreren definierbaren Richtungen ein im zu beurteilenden und eventuell geschädigten Stamm-Querschnitt (4) jeweils ermitteltes Widerstandsmoment durch ein ermitteltes Widerstandsmoment desselben Stamm-Querschnitts (4) in einem angenommenen ungeschädigten Zustand dividiert wird; und
   - Zusammenführen der berechneten prozentualen relativen richtungsabhängigen Tragfähigkeiten zur Bewertung der Bruchsicherheit des Baums (1),
   wobei bei der Bewertung der Bruchsicherheit ein Vergleich des zu beurteilenden Stamm-Querschnitts (4) mit einem intakten Stamm-Querschnitt (4) am gleichen Stamm des Baums (1) erfolgt, um eine relative Grundsicherheit RGS zu bestimmen, und
   wobei bei der Bewertung oder Berechnung der Bruchsicherheit ein Quotient aus

$$D^3 / H^3$$

   berücksichtigt wird, wobei D der Durchmesser des Stamm-Querschnitts (4) und H die Höhe des Baums (1) ist.

2. Verfahren nach Anspruch 1, **dadurch gekennzeichnet, dass** das Erfassen des größten und des kleinsten Durchmessers ein Messen des größten und des kleinsten Durchmessers am Baum (1) oder ein Beschaffen des größten und des kleinsten Durchmessers aus einer Datenbank umfasst.

3. Verfahren nach Anspruch 1 oder 2, **dadurch gekennzeichnet, dass** das Berechnen der Tragfähigkeit entlang mehreren Richtungen in vorgebbaren Winkelabständen entlang eines gesamten Umfangs des Stamm-Querschnitts (4) erfolgt.

4. Verfahren nach einem der Ansprüche 1 bis 3, **dadurch gekennzeichnet, dass** die Tragfähigkeit in einer jeweiligen Richtung über Widerstandsmomente mittels einer Integration über eine Querschnittsfläche des Stamm-Querschnitts (4) berechnet wird.

5. Verfahren nach einem der Ansprüche 1 bis 4, **dadurch gekennzeichnet, dass** bei der Bewertung der Bruchsicherheit ein Maximalwert einer richtungsabhängigen relativen - vorzugsweise prozentualen - Schwächung der Tragfähigkeit berücksichtigt wird, um eine relative Resttragfähigkeit RQT zu bestimmen.

6. Verfahren nach einem der Ansprüche 1 bis 5, **dadurch gekennzeichnet, dass** bei der Bewertung der Bruchsicherheit eines Stamm-Querschnitts (4) eine Grundsicherheit des Stamm-Querschnitts (4) berücksichtigt wird.

7. Verfahren nach einem einem der Ansprüche 1 bis 6, **dadurch gekennzeichnet, dass** die Bewertung der Bruchsicherheit des zu berteilenden geschädigten Querschnitts (4) durch alloemtrisch-rechnerischen Vergleich mit einem intakten Querschnitt (4) desselben Stamms (3) erfolgt, indem $(Dd/Di)^3 * (Li/Ld)$ berechnet wird.

8. Verfahren nach einem der Ansprüche 1 bis 7, **dadurch gekennzeichnet, dass** bei der Bewertung der Bruchsicherheit eine Reduktion einer Höhe H und/oder ein altersbedingter Rückgang einer Höhe H des Baums (1) berücksichtigt wird, wobei sich die Bruchsicherheit hierbei um den Faktor

$$(H_{vorher})^3 / (H_{neu})^3 \quad und/oder \quad (H_{max})^3 / (H_{aktuell})^3$$

   erhöht.

**9.** Verfahren nach einem der Ansprüche 1 bis 8, **dadurch gekennzeichnet, dass** bei der Bewertung der Bruchsicherheit ein altersbedingter radialer Zuwachs des Durchmessers D des Stamm-Querschnitts (4) des Baums (1) berücksichtigt wird, wobei sich die Bruchsicherheit hierbei um den Faktor

$$(D_{aktuell})^3 \, / \, (D_{Hmax})^3$$

erhöht.

**10.** Vorrichtung zur Durchführung des Verfahrens zur Ermittlung der Bruchsicherheit eines Baums (1) nach einem der Ansprüche 1 bis 9, umfassend:

- Erfassungsmittel zum Erfassen mindestens eines größten und eines kleinsten Durchmessers eines zu beurteilenden Stamm-Querschnitts (4) eines Baums (1) sowie einer Form mit eventuell vorhandenen Schädigungen des Stamm-Querschnitts (4);
- Rechenmittel zum Berechnen - ausgehend von einer durch einen geometrischen Schwerpunkt des Stamm-Querschnitts (4) verlaufenden Achse - der neutralen Achse - einer prozentualen relativen richtungsabhängigen Tragfähigkeit in mehreren definierbaren Richtungen über jeweils dem Stamm-Querschnitt (4) zugehörige und gemäß Anisotropie des Materials spezifisch ermittelte Widerstandsmomente, wobei zur Berechnung einer jeweiligen prozentualen relativen richtungsabhängigen Tragfähigkeit für jede der mehreren definierbaren Richtungen ein im zu beurteilenden und eventuell geschädigten Stamm-Querschnitt (4) jeweils ermitteltes Widerstandsmoment durch ein ermitteltes Widerstandsmoment desselben Stamm-Querschnitts (4) in einem angenommenen ungeschädigten Zustand dividiert wird;
- Zusammenführungsmittel zum Zusammenführen der berechneten prozentualen relativen richtungsabhängigen Tragfähigkeiten zur Bewertung der Bruch-sicherheit des Baums (1),
- Mittel, um bei der Bewertung der Bruchsicherheit einen Vergleich des zu beurteilenden Stamm-Querschnitts (4) mit einem intakten Stamm-Querschnitt (4) am gleichen Stamm des Baums (1) durchzuführen, um eine relative Grundsicherheit RGS zu bestimmen; und
- Mittel zur Berücksichtigung eines Quotienten aus

$$D^3 \, / \, H^3$$

bei der Bewertung oder Berechnung der Bruchsicherheit, wobei D der Durchmesser des Stamm-Querschnitts (4) und H die Höhe des Baums (1) ist.

**Claims**

**1.** Method for establishing the break resistance of a tree (1), comprising the following steps:

- determining a trunk cross-section (4) of the tree (1) which is intended to be assessed;
- detecting at least one largest and one smallest diameter in the trunk cross-section (4) and the shape thereof with any damage to the trunk cross-section (4) which may be present,
- calculating - based on an axis which extends through a geometric centre of gravity of the trunk cross-section (4) - the neutral axis - a percentage relative direction-dependent load-bearing capacity in a plurality of definable directions via resistance torques which are associated in each case with the trunk cross-section (4) and which are specifically established in accordance with the anisotropy of the material, wherein in order to calculate a respective percentage relative direction-dependent load-bearing capacity for each of the plurality of definable directions a resistance torque which is established in each case in a trunk cross-section (4) which is intended to be assessed and which may be damaged is divided by an established resistance torque of the same trunk cross-section (4) in an assumed undamaged state; and
- combining the calculated percentage relative direction-dependent load-bearing capacities in order to evaluate the break resistance of the tree (1),

wherein during the evaluation of the break resistance a comparison of the trunk cross-section (4) which is intended to be assessed with an intact trunk cross-section (4) on the same trunk of the tree (1) is carried out in order to determine a relative basic resistance RGS, and

wherein during the evaluation or calculation of the break resistance a quotient of

$$D^3/H^3$$

is taken into consideration, wherein D is the diameter of the trunk cross-section (4) and H is the height of the tree (1).

2. Method according to claim 1, **characterised in that** the detection of the largest and the smallest diameter involves a measurement of the largest and the smallest diameter on the tree (1) or obtaining the largest and the smallest diameter from a database.

3. Method according to claim 1 or 2, **characterised in that** the calculation of the load-bearing capacity is carried out in a plurality of directions at predeterminable angular spacings along an entire circumference of the trunk cross-section (4).

4. Method according to any one of claims 1 to 3, **characterised in that** the load-bearing capacity is calculated in a respective direction via resistance torques by means of an integration over a cross-sectional surface-area of the trunk cross-section (4).

5. Method according to any one of claims 1 to 4, **characterised in that** during the evaluation of the break resistance a maximum value of a direction-dependent relative - preferably percentage - weakening of the load-bearing capacity is taken into consideration in order to determine a relative residual load-bearing capacity RQT.

6. Method according to any one of claims 1 to 5, **characterised in that** during the evaluation of the break resistance of a trunk cross-section (4) a basic resistance of the trunk cross-section (4) is taken into consideration.

7. Method according to any one of claims 1 to 6, **characterised in that** the evaluation of the break resistance of the damaged cross-section (4) which is intended to be assessed by means of allometric/mathematical comparison with an intact cross-section (4) of the same trunk (3) is carried out by $(Dd/Di)^3 {}^* (Li/Ld)$ being calculated.

8. Method according to any one of claims 1 to 7, **characterised in that** during the evaluation of the break resistance a reduction of a height H and/or an age-related decrease of a height H of the tree (1) is taken into consideration, wherein the break resistance increases in this instance by the factor

$$(H_{vorher})^3 \ / \ (H_{neu})^3 \ \text{and/or} \ (H_{max})^3 \ / \ (H_{aktuell})^3.$$

9. Method according to any one of claims 1 to 8, **characterised in that** during the evaluation of the break resistance an age-related radial growth of the diameter D of the trunk cross-section (4) of the tree (1) is taken into consideration, wherein the break resistance increases in this instance by the factor

$$(D_{aktuell})^3 \ / \ (D_{Hmax})^3.$$

10. Apparatus for carrying out the method for establishing the break resistance of a tree (1) according to any one of claims 1 to 9, comprising:

   - detection means for detecting at least one largest and one smallest diameter of a trunk cross-section (4) of a tree (1) which is intended to be assessed and a shape with any damage of the trunk cross-section (4) which may be present;
   - calculation means for calculating - starting from an axis which extends through a geometric centre of gravity of the trunk cross-section (4) - the neutral axis - a percentage relative direction-dependent load-bearing capacity in a plurality of definable directions via resistance torques which are associated in each case with the trunk cross-section (4) and which are specifically established in accordance with the anisotropy of the material, wherein in order to calculate a respective percentage relative direction-dependent load-bearing capacity for each of the plurality of definable directions a resistance torque which is established in each case in the trunk cross-section (4) which is intended to be assessed and which may be damaged is divided by an established resistance torque of the same trunk cross-section (4) in an assumed undamaged state;
   - combination means for combining the calculated percentage relative direction-dependent load-bearing capacities in order to evaluate the break resistance of the tree (1),
- means in order during the evaluation of the break resistance to carry out a comparison of the trunk cross-section (4) which is intended to be assessed with an intact trunk cross-section (4) on the same trunk of the tree (1) in order to determine a relative basic resistance RGS; and means for considering a quotient of

$$D^3/H^3$$

during the evaluation or calculation of the break resistance, wherein D is the diameter of the trunk cross-section (4) and H is the height of the tree (1).

**Revendications**

1. Procédé de détermination de la résistance à la rupture d'un arbre (1), comprenant les étapes suivantes :

   - définition d'une section transversale de tronc (4) de l'arbre (1) à évaluer ;
   - mesure d'au moins le plus grand et le plus petit diamètre dans la section transversale de tronc (4) ainsi que d'une forme avec d'éventuels endommagements de la section transversale de tronc (4) ;
   - calcul - à partir d'un axe passant par un centre de gravité géométrique de la section transversale de tronc (4) - l'axe neutre - d'une capacité de charge relative en fonction de la direction en pourcentage, dans plusieurs directions définies, sur des couples de résistance correspondant respectivement à la section transversale du tronc (4) et déterminés spécifiquement selon l'anisotropie du matériau, dans lequel, pour le calcul d'une capacité de charge relative en fonction de la direction en pourcentage, pour chacune des directions définies, un couple de résistance, déterminé respectivement dans la section transversale du tronc (4) à évaluer et éventuellement endommagée, est divisé par un couple de résistance déterminé de la même section transversale de tronc (4) dans un état supposé non endommagé ; et
   - regroupement des capacités de charge relatives en fonction de la direction, en pourcentage, calculées pour l'évaluation de la résistance à la rupture de l'arbre (1) dans lequel, lors de l'évaluation de la résistance à la rupture, a lieu une comparaison

   de la section transversale de tronc (4) à évaluer avec une section transversale de tronc intacte (4) sur le même tronc de l'arbre (1), afin de déterminer une résistance de base relative RGS et
   dans lequel, lors de l'évaluation ou du calcul de la résistance à la rupture, un rapport $D^3/H^3$ est pris en compte, dans lequel D est le diamètre de la section transversale du tronc (4) et H est la hauteur de l'arbre (1).

2. Procédé selon la revendication 1, **caractérisé en ce que** la mesure du plus grand diamètre et du plus petit diamètre comprend une mesure du plus grand diamètre et du plus petit diamètre sur l'arbre (1) ou une récupération du plus grand diamètre et du plus petit diamètre dans une base de données.

3. Procédé selon la revendication 1 ou 2, **caractérisé en ce que** le calcul de la capacité de charge dans plusieurs directions a lieu à des intervalles angulaires prédéterminés le long d'une circonférence totale de la section transversale du tronc (4).

4. Procédé selon l'une des revendications 1 à 3, **caractérisé en ce que** la capacité de charge dans une direction respective est calculée sur des couples de résistance au moyen d'une intégration sur une surface de section transversale de la section transversale de tronc (4).

5. Procédé selon l'une des revendications 1 à 4, **caractérisé en ce que**, lors de l'évaluation de la résistance à la rupture, une valeur maximale d'un affaiblissement relatif en fonction de la direction - de préférence en pourcentage - de la capacité de charge est pris en compte, afin de déterminer une capacité de charge résiduelle RQT.

6. Procédé selon l'une des revendications 1 à 5, **caractérisé en ce que**, lors de l'évaluation de la résistance à la rupture d'une section transversale de tronc (4), une résistance de base de la section transversale de tronc (4) est prise en compte.

7. Procédé selon l'une des revendications 1 à 6, **caractérisé en ce que** l'évaluation de la résistance à la rupture de la section transversale (4) endommagée à évaluer est effectuée à l'aide d'une comparaison allométrique-mathématique avec une section transversale intacte (4) du même tronc (3), en calculant Dd/Di$^3$ * (Li/Ld).

8. Procédé selon l'une des revendications 1 à 7, **caractérisé en ce que**, lors de l'évaluation de la résistance à la rupture, une réduction d'une hauteur H et/ou une diminution, due à l'âge, d'une hauteur H de l'arbre (1) est prise en compte, dans lequel la résistance à la rupture est augmentée du facteur

$$(H_{vorher})^3 \, / \, (H_{neu})^3 \text{ et/ou } (H_{max})^3 \, / \, (H_{aktuell})^3.$$

9. Procédé selon l'une des revendications 1 à 8, **caractérisé en ce que**, lors de l'évaluation de la résistance à la rupture, une croissance radiale, due à l'âge, du diamètre D de la section transversale du tronc (4) de l'arbre (1) est prise en compte, dans lequel la résistance à la rupture est augmentée du facteur

$$(D_{aktuell})^3 \, / \, (D_{hmax})^3.$$

10. Dispositif pour l'exécution du procédé de détermination de la résistance à la rupture d'un arbre (1) selon l'une des revendications 1 à 9, comprenant :

  - des moyens de mesure pour la mesure d'au moins le plus grand et le plus petit diamètre d'une section transversale de tronc (4) d'un arbre (1) ainsi que d'une forme avec d'éventuels endommagements de la section transversale de tronc (4) ;
  - des moyens de calcul pour le calcul - à partir d'un axe passant par un centre de gravité géométrique de la section transversale de tronc (4) - l'axe neutre - d'une capacité de charge relative en fonction de la direction en pourcentage, dans plusieurs directions définies, sur des couples de résistance correspondant respectivement à la section transversale du tronc (4) et déterminés spécifiquement selon l'anisotropie du matériau, dans lequel, pour le calcul d'une capacité de charge relative en fonction de la direction en pourcentage, pour chacune des directions définies, un couple de résistance, déterminé respectivement dans la section transversale du tronc (4) à évaluer et éventuellement endommagée, est divisé par un couple de résistance déterminé de la même section transversale de tronc (4) dans un état supposé non endommagé ;
  - des moyens de regroupement pour le regroupement des capacités de charge relatives en fonction de la direction, en pourcentage, calculées pour l'évaluation de la résistance à la rupture de l'arbre (1),
  - des moyens pour effectuer, lors de l'évaluation de la résistance à la rupture, une comparaison de la section transversale de tronc (4) à évaluer avec une section transversale de tronc (4) intacte sur le même tronc de l'arbre (1), afin de déterminer une résistance de base relative RGS ; et
  - des moyens pour la prise en compte d'un rapport

$$D^3 \, / \, H^3$$

lors de l'évaluation ou du calcul de la résistance à la rupture, dans lequel D est de diamètre de la section transversale de tronc (4) et H est la hauteur de l'arbre (1).

EP 4 047 363 B1

Fig. 1

Fig. 2

Fig. 3

Fig. 4

Fig. 5

EP 4 047 363 B1

Fig. 6

Fig. 7

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SPATZ, H.C. ; NIKLAS, K.J.** Modes of failure in tubular plant organs. *Am. J. Bot.,* 2013, vol. 100 (2), 332-336

- **KOIZUMI AKIO et al.** Evaluation of the section modulus for tree-stem cross sections of irregular shape. *JOURNAL OF WOOD SCIENCE,* 01. Juni 2006, vol. 52 (3), ISSN 1435-0211, 213-219